# EUROPÄISCHE PATENTSCHRIFT

(11) **EP 0 482 410 B1**
(45) Veröffentlichungstag und Bekanntmachung des Hinweises auf die Patenterteilung: **05.02.1997**
(21) Anmeldenummer: 91117031.4
(22) Anmeldetag: 07.10.1991
(51) Int. Cl.: A01N 37/44, A01N 37/46, C07C 237/24, C07C 237/04, C07C 229/48, C07C 327/14, C07C 233/52, A01N 43/40, C07D 213/30

(54) **Antimikrobielle Mittel sowie substituierte 2-Cyclohexan-1-yl-amin-Derivate und deren Herstellung**
Anti-microbial compounds, including substituted 2-cyclohexyl-amine derivatives and their preparation
Composés anti-microbiennes et dérivés de cyclohexylamines 2-substituées et leur préparation

(30) Priorität: 20.10.1990 DE 4033415
(43) Veröffentlichungstag der Anmeldung: 29.04.1992
(73) Patentinhaber: BAYER AG, 51368 Leverkusen (DE)
(72) Erfinder: Kunisch, Franz, Dr., W-5068 Odenthal (DE); Babczinski, Peter, Dr., W-5600 Wuppertal 1 (DE); Arlt, Dieter, Dr., W-5000 Köln 80 (DE); Brandes, Wilhelm, Dr., W-5653 Leichlingen 1 (DE); Dehne, Heinz-Wilhelm, Dr., W-4019 Monheim (DE); Dutzmann, Stefan, Dr., W-4010 Hilden 1 (DE); Plempel, Manfred, Dr., W-5657 Haan (DE)

(56) Entgegenhaltungen:
- EP-A- 0 005 782
- EP-A- 0 376 072
- DE-A- 1 935 458
- FR-A- 820 736
- FR-A- 2 313 023
- US-A- 3 510 492
- US-A- 4 554 277
- US-A- 4 673 631
- CHEMICAL ABSTRACTS, Band 94, Nr. 25, 22. Juni 1981, Seite 374, Zusammenfassung 206197u, Columbus, Ohio, US; JO CUNNINGHAM et al.: "The radioautographical localization in the vertebrate retina of [3H]-(+)-cis-aminocyclohexane carboxylic acid (ACHC); a selective inhibitor of neuronal GABA transport", && EXP. EYE RES. 1981,32(4),445-50
- BULLETIN OF THE CHEMICAL SOCIETY OF JAPAN, Band 43, Nr. 7, Juli 1970, Seiten 2230-2233, JP; H. NOHIRA et al.: "The resolution and rotations of trans-2-aminocyclo-hexanecarboxylic acids and derivatives"

## Beschreibung

Die vorliegende Erfindung betrifft die Verwendung von teilweise bekannten substituierten 2-Cyclohexan-1-yl-amin-Derivaten als antimikrobielle Mittel im Pflanzenschutz, vor allem als Fungizide sowie neue substituierte 2-Cyclohexan-1-yl-amin-Derivate und mehrere Verfahren zu ihrer Herstellung.

2-Cyclohexan-1-yl-amin Derivate sind bekannt und werden z.B. beschrieben in US-3 510 492, FR-2 313 023, US 4 673 631, Bulletin of the Chemical Society of Japan Band 43, S.2230-3 (1970)

Es ist bereits bekannt, daß bestimmte Tetrahydrophthalimide, wie beispielsweise cis-N-[(Trichlormethyl)thio]-4-cyclohexen-1,2-dicarboimid, fungizide Eigenschaften aufweisen (vgl. z.B. Science, (Washington) 115, 84 (1952); US 2 553 770; EP-376 072).

Die Wirkung dieser Verbindungen ist jedoch insbesondere bei niedrigen Aufwandmengen und -konzentrationen nicht immer in allen Anwendungsbereichen völlig zufriedenstellend.

Es ist weiter bereits bekannt, daß 2-Cycloalkenylamin-Derivate und ihre Salze, wie beispielsweise N-2-Cyclohexen-1-yl-2,2-dimethyl-propionamid fungizide Eigenschaften besitzen (vgl. EP-OS 0 128 006).

Es wurde nun gefunden, daß die teilweise bekannten substituierten 2-Cyclohexan-1-yl-amin-Derivate der Formel (Ia) 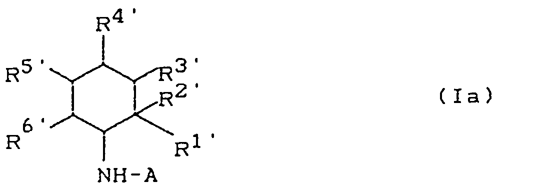 in welcher
- R^{1'}: für Wasserstoff, Halogen oder geradkettiges oder verzweigtes Alkyl mit 1 bis 6 Kohlenstoffatomen steht,
- R^{2'}: für Formyl, geradkettiges oder verzweigtes Hydroxyalkyl mit 1 bis 8 Kohlenstoffatomen im Alkylteil oder für einen der Reste 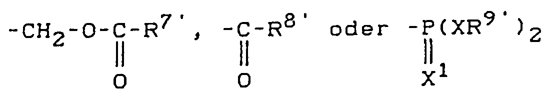 steht,
- R^{3'}, R^{4'}, R^{5'} und R^{6'}: gleich oder verschieden sind und jeweils für Wasserstoff, jeweils geradkettiges oder verzweigtes Alkyl oder Alkoxy mit 1 bis 8 Kohlenstoffatomen, Alkoxyalkyloxy mit jeweils 1 bis 8 Kohlenstoffatomen in den einzelnen Alkylteilen, für jeweils im Arylteil unsubtituiertes oder einfach bis fünffach, gleich oder verschieden substituiertes Aryl oder Aralkyl mit jeweils 6 bis 10 Kohlenstoffatomen im Arylteil und gegebenenfalls 1 bis 4 Kohlenstoffatomen im Alkylteil stehen, wobei als Arylsubstituenten infrage kommen: Halogen, Nitro, Cyano, Amino, C₁-C₄-Alkyl, C₁-C₄-Alkoxy, C₁-C₄-Alkylthio, Halogen-(C₁-C₄)-alkyl, Halogen-(C₁-C₄)-alkoxy, Halogen-(C₁-C₄)-alkylthio mit jeweils 1 bis 9 gleichen oder verschiedenen Halogenatomen und Di-(C₁-C₄)-alkylamino,
weiterhin für eine unsubstituierte oder einfach bis dreifach, gleich oder verschieden substituierte und gegebenenfalls über eine Methylengruppe gebundene heterocyclische 5- oder 6-gliedrige Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Triazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl stehen, wobei als Substituen für den Heteroyclus jeweils infrage kommen: Halogen, Nitro, Cyano, Amino, C₁-C₄-Alkyl, C₁-C₄-Alkoxy oder C₁-C₄-Alkylthio, Halogen-(C₁-C₄-alkyl, Halogen-(C₁-C₄)-alkoxy, Halogen-(C₁-C₄)-alkylthio mit jeweils 1 bis 9 gleichen oder verschiedenen Halogenatomen und Di-(C₁-C₄)-alkylamino,
wobei mindestens zwei der Reste R^{3'}, R^{4'}, R^{5'} oder R^{6'} für Wasserstoff stehen.
- R^{7'}: für jeweils geradkettiges oder verzweigtes Alkyl der Alkoxy mit 1 bis 6 Kohlenstoffatomen steht,
- R^{8'}: für Hydroxy, geradkettiges oder verzweigtes Hydroxyalkyloxy mit 1 bis 8 Kohlenstoffatomen,
geradkettiges oder verzweigtes Halogenalkyloxy mit 1 bis 8 Kohlenstoffatomen und 1 bis 17 gleichen oder verschiedenen Halogenatomen, jeweils unsubstituiertes oder einfach bis mehrfach, gleich oder verschieden durch Halogen substituiertes Alkenylalkyloxy, Alkinylalkyloxy und Cycloalkyloxy mit 3 bis 6 Kohlenstoffatomen, jeweils geradkettiges oder verzweigtes Alkoxy oder Alkylthio mit 1 bis 6 Kohlenstoffatomen, geradkettiges oder verzweigtes Alkoxyalkyloxy mit jeweils 1 bis 6 Kohlenstoffatomen im Alkoxy- oder Alkylteil, jeweils im Arylteil unsubstituiertes oder einfach bis fünffach, gleich oder verschieden substituiertes Aryloxy, Arylthio, Aralkyl oder Aralkyloxy mit jeweils 6 bis 10 Kohlenstoffatomen im Arylteil und gegebenenfalls 1 bis 8 Kohlenstoffatomen im Alkylteil steht, wobei als Arylsubstituenten die oben aufgeführten Arylsubstituenten in Frage kommen oder für eine1Gruppe -O-Z-NR^{10'}R^{11'},
-NR^{10'}R^{11'} oder -OM steht,
- R^{9'}: für Wasserstoff oder geradkettiges oder verzweigtes Alkyl mit 1 bis 6 Kohlenstoffatomen steht,
- R^{10'} und R^{11'}: gleich oder verschieden sind und für Wasserstoff, geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen stehen,
wobei als Arylsubstituenten die oben aufgeführten Arylsubstituenten in Frage kommen,
- M: für Wasserstoff oder für ein Äquivalent eines entsprechenden Alkalimetall-, Erdalkalimetall- oder Ammoniumkations steht und
- Z: für eine geradkettige oder verzweigte Alkylkette mit 1 bis 8 Kohlenstoffatomen steht
- A: für Wasserstoff oder eine Aminoschutzgruppe steht und
- X und X¹: gleich oder verschieden sind und für Sauerstofff oder Schwefel stehen,
sowie deren Säureadditions-Salze und Metallsalz-Komplexe starke biologische Eigenschaften aufweisen.

Die Verbindungen der Formel (Ia) enthalten 1 bis 4 Chiralitätszentren und können somit in verschiedenen Enantiomeren- und Diastereomerengemischen vorliegen, die gegebenenfalls in üblicher Art und Weise getrennt werden können. Die Verwendung sowohl der reinen Enantiomeren und Diastereomeren, als auch die der Gemische werden ebenfalls erfindungsgemäß beansprucht.

Im folgenden wird der Einfachheit halber stets von der Verwendung von Verbindungen der Formel (Ia) gesprochen, obwohl sowohl die reinen Verbindungen, als auch die Gemische mit unterschiedlichen Anteilen an isomeren, enantiomeren und diastereomeren Verbindungen gemeint sind.

Überraschenderweise zeigen die teilweise bekannten substituierten 2-Cyclohexan-1-yl-amin-Derivate der Formel (Ia) sowie deren Säureadditions-Salze und Metallsalz-Komplexe bei entsprechenden Anwendungskonzentrationen ausgezeichnete fungizide Eigenschaften. Zusätzlich zeigen die teilweise bekannten substituierten 2-Cyclohexan-1-yl-amin-Derivate der Formel (Ia) bei entsprechenden Anwendungskonzentrationen auch sehr gute antimikrobielle Eigenschaften.

Die erfindungsgemäß zu verwendenden substituierten 2-Cyclohexan-1-yl-amin-Derivate sind durch die Formel (Ia) allgemein definiert.

Im folgenden bedeutet in den allgemeinen Formeln, falls nicht anders definiert:

Alkyl - geradkettiges oder verzweigtes Alkyl mit 1 bis 8, vorzugsweise 1 bis 6, insbesondere 1 bis 4 Kohlenstoffatomen. Beispielhaft und vorzugsweise seien gegebenenfalls substituiertes Methyl, Ethyl, n.- und i.-Propyl, n-, i-, s- und t-Butyl, genannt.

Alkoxy - unsubstituiertes oder substituiertes, geradkettiges oder verzweigtes Alkoxy mit 1 bis 8, vorzugsweise 1 bis 6, insbesondere 1 bis 4 Kohlenstoffatomen. Beispielhaft und vorzugsweise seien gegebenenfalls substituiertes Methoxy, Ethoxy, n.- und i.-Propoxy und n-, i-, s- und t-Butoxy genannt.

Aryl - vorzugsweise unsubstituiertes oder substituiertes Phenyl oder Naphtyl, insbesondere Phenyl.

Aralkyl und Aralkoxy - unsubstituiertes oder im Arylteil und/oder Alkylteil substituiertes Aralkyl bzw. Aralkoxy mit vorzugsweise 6 oder 10, insbesondere 6 Kohlenstoffatomen im Arylteil (vorzugsweise Phenyl oder Naphtyl, insbesondere Phenyl) und vorzugsweise 1 bis 8, insbesondere 1 bis 6 Kohlenstoffatomen im Alkylteil, wobei der Alkylteil geradkettig oder verzweigt sein kann. Beispielhaft und vorzugsweise seien gegebenenfalls substituiertes Benzyl und Phenylethyl bzw. Benzyloxy und Phenylethyloxy genannt.

Unsubstituierte oder substituierte heterocyclische Reste bedeuten in den allgemeinen Formeln heteroaromatische 5-6-gliedrige Ringe mit vorzugsweise 1 bis 3, insbesondere 1 oder 2 gleichen oder verschiedenen Heteroatomen. Als Heteroatome stehen Sauerstoff, Schwefel oder Stickstoff. Beispielhaft und vorzugsweise seien Pyrrolidinyl, Piperidinyl, Furyl, Thienyl, Pyrazolyl, Imidazolyl, 1,2,3- und 1,2,4-Triazolyl, Oxazolyl, Isoxazolyl, Thiazolyl, Isothiazolyl, 1,3,4-und 1,2,4-Oxadiazolyl, Azepinyl, Pyrrolyl, Isopyrrolyl, Pyridyl, Pyridazinyl, Pyrimidinyl, Pyrazinyl und 1,2,3-, 1,2,4-, 1,2,5- und 1,3,4-Thiadiazolyl genannt.

Halogen bedeutet in den allgemeinen Formeln vorzugsweise Fluor, Chlor, Brom und Jod, insbesondere Fluor, Chlor und Brom und besonders bevorzugt Fluor und Chlor.

Die gegebenenfalls substituierten Reste der allgemeinen Formeln können einen oder mehrere, vorzugsweise 1 bis 3, insbesondere 1 oder 2 gleiche oder verschiedene Substituenten tragen. Als Substituenten seien beispielhaft und vorzugsweise aufgeführt:

Alkyl mit vorzugsweise 1 bis 4, insbesondere 1 oder 2 Kohlenstoffatomen, wie Methyl, Ethyl, n.- und i.-Propyl und n.-, i.- und t.-Butyl; Alkoxy mit vorzugsweise 1 bis 4, insbesondere 1 oder 2 Kohlenstoffatomen, wie Methoxy, Ethoxy, n.- und i.-Propyloxy und n.-, i.-, sec.- und t.-Butyloxy; Alkylthio mit vorzugsweise 1 bis 4, insbesondere 1 oder 2 Kohlenstoffatomen, wie Methylthio, Ethylthio, n.- und i.-Propylthio und n.-, i.-, sec.- und t.-Butylthio; Halogenalkyl, Halogenalkoxy und Halogenalkylthio mit vorzugsweise 1 bis 4, insbesondere 1 oder 2 Kohlenstoffatomen und vorzugsweise 1 bis 9, insbesondere 1 bis 5 Halogenatomen, wobei die Halogenatome gleich oder verschieden sind und als Halogenatome, vorzugsweise Fluor, Chlor oder Brom, insbesondere Fluor stehen, wie Trifluormethyl, Trifluormethoxy und Trifluormethylthio; Hydroxy; Halogen, vorzugsweise Fluor, Chlor, Brom und Jod, insbesondere Fluor, Chlor und Brom; Cyano; Nitro; Amino; Dialkylamino mit vorzugsweise 1 bis 4, insbesondere 1 oder 2 Kohlenstoffatomen je Alkylgruppe, wie Methyl-ethyl-amino, und Methyl-n.-butylamino; Carboxyl.

Bevorzugt erfindungsgemäß zu verwendende Verbindungen sind auch Additionsprodukte aus Säuren und denjenigen substituierten 2-Cyclohexan-1-yl-amin-Derivaten der Formel (Ia) in denen R¹, R², R³ und R⁴ diejenigen Bedeutungen haben, die im Zusammenhang mit der Beschreibung der erfindungsgemäß zu verwendenden Stoffe bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Trifluoressigsäure, Maleinsäure, Bernsteinsäure, Fumarsäure, Weinsäure, Zitronensäure, Salicylsäure, Sorbinsäure und Milchsäure, Ölsäure, Stearinsäure, gegebenenfalls einfach bis mehrfach durch Nitro oder Halogen substituierte Benzoesäure, Gluconsäure, Ascorbinsäure, Äpfelsäure, Sulfamidsäure, Sulfonsäuren, wie z.B. p-Toluolsulfonsäure, 1,5-Naphthalindisulfonsäure und Methansulfonsäure, sowie Imide, wie z.B. Phthalimid, Saccharin und Thiosaccharin.

Außerdem bevorzugt erfindungsgemäß zu verwendende Verbindungen sind auch Additionsprodukte aus Salzen von Metallen der I., II. und III. Hauptgruppe sowie des Zinns, sowie ferner Salze von Metallen der I., II., VII, und VIII. Nebengruppe des Periodensystems der Elemente und denjenigen substituierten 2-Cyclohexan-1-yl-amin-Derivaten der Formel (Ia), in denen R¹, R², R³ und R⁴ die Bedeutungen haben, die bereits im Zusammenhang mit der Beschreibung der erfindungsgemäß zu verwendenden Stoffe der Formel (Ia) vorzugsweise für diese Substituenten genannt wurden.

Hierbei sind Salze des Kupfers, Zinks, Mangans, Magnesiums, Calciums, Zinns, Eisens, Cobalts und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte Reaktion an anderen Stellen des Moleküls durchgeführt worden ist. Typisch für solche Gruppen sind insbesondere unsubstituierte oder substituierte Acyl-, Aryl- z.B. DNP (2,4-Dinitrophenyl), Aralkoxymethyl- z.B. BOM (N-(benzyloxy)methyl) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1 - 20, insbesondere 1 - 8 Kohlenstoffatomen. Der Ausdruck "Acylgruppe" ist im Zusammenhang mit der vorliegenden Erfindung in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA (Phenoxyacetyl); Alkoxycarbonyl wie Methoxycarbonyl, Ethyoxycarbonyl, 2,2,2-Trichlorethoxycarbonyl, BOC (tert.-Butoxycarbonyl), 2-Iodethoxycarbonyl; Aralkyloxycarbonyl wie CBZ ("Carbobenzoxy") und 4-Methoxybenzyloxycarbonyl. Bevorzugte Aminoschutzgruppen sind Benzyl, Acetyl, Methoxycarbonyl, Allyloxycarbonyl, Trichlorethyloxycarbonyl, (±)-Menthyloxycarbonyl, tert-Butoxycarbonyl und Benzyloxycarbonyl.

Ein weiterer Gegenstand der Anmeldung sind neue substituierte 2-Cyclohexan-1-yl-amin-Derivate der Formel (Ia) 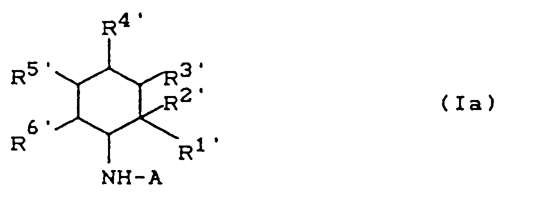 in welcher
- R^{1'}: für Wasserstoff, Halogen oder geradkettiges oder verzweigtes Alkyl mit 1 bis 6 Kohlenstoffatomen steht,
- R^{2'}: für Formyl, geradkettiges oder verzweigtes Hydroxyalkyl mit 1 bis 8 Kohlenstoffatomen im Alkylteil, oder für einen der Reste 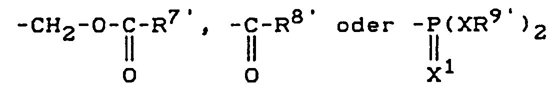 steht,
- R^{3'}, R^{4'}, R^{5'} und R^{6'}: gleich oder verschieden sind und jeweils für Wasserstoff, jeweils geradkettiges oder verzweigtes Alkyl oder Alkoxy mit 1 bis 8 Kohlenstoffatomen, Alkoxyalkyloxy mit jeweils 1 bis 8 Kohlenstoffatomen in den einzelnen Alkylteilen, für jeweils im Arylteil unsubstituiertes oder einfach bis fünffach, gleich oder verschieden substituiertes Aryl oder Aralkyl mit jeweils 6 bis 10 Kohlenstoffatomen im Arylteil und gegebenenfalls 1 bis 4 Kohlenstoffatomen im Alkylteil stehen, wobei als Arylsubstituenten in Frage kommen: Halogen, Nitro, Cyano, Amino, C₁-C₄-Alkyl, C₁-C₄-Alkoxy, C₁-C₄-Alkylthio, Halogen-(C₁-C₄)-alkyl, Halogen-(C₁-C₄)-alkoxy, Halogen-(C₁-C₄)-alkylthio mit jeweils 1 bis 9 gleichen oder verschiedenen Halogenatomen und Di-(C₁-C₄)-alkylamino, weiterhin für eine unsubstituierte oder einfach bis dreifach, gleich oder verschieden substituierte und gegebenenfalls über eine Methylengruppe gebundene heterocyclische 5- oder 6-gliedrige Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl stehen, wobei als Substituenten für den Heterocyclus jeweils infrage kommen: Halogen, Nitro, Cyano, Amino, C₁-C₄-Alkyl, C₁-C₄-Alkoxy oder C₁-C₄-Alkylthio, Halogen-(C₁-C₄)-alkyl, Halogen-(C₁-C₄)-alkoxy oder Halogen-(C₁-C₄)-alkylthio mit jeweils 1 bis 9 gleichen oder verschiedenen Halogenatomen und Di-(C₁-C₄)-alkylamino,
wobei mindestens zwei der Reste R^{3'}, R^{4'}, R^{5'} oder R^{6'} für Wasserstoff stehen,
- R^{7'}: für jeweils geradkettiges oder verzweigtes Alkyl oder Alkoxy mit 1 bis 6 Kohlenstoffatomen steht,
- R^{8'}: für Hydroxy, geradkettiges oder verzweigtes Hydroxyalkyloxy mit 1 bis 8 Kohlenstoffatomen, geradkettiges oder verzweigtes Halogenalkyloxy mit 1 bis 8 Kohlenstoffatomen und 1 bis 17 gleichen oder verschiedenen Halogenatomen, jeweils unsubstituiertes oder einfach bis mehrfach, gleich oder verschieden durch Halogen substituiertes Alkenylalkyloxy, Alkinylalkyloxy und Cycloalkyloxy mit 3 bis 6 Kohlenstoffatomen, jeweils geradkettiges oder verzweigtes Alkoxy oder Alkylthio mit 1 bis 6 Kohlenstoffatomen, geradkettiges oder verzweigtes Alkoxyalkyloxy mit jeweils 1 bis 6 Kohlenstoffatomen im Alkoxy- oder Alkylteil, jeweils im Arylteil unsubstituiertes oder einfach bis fünffach, gleich oder verschieden substituiertes Aryloxy, Arylthio, Aralkyl oder Aralkyloxy mit jeweils 6 bis 10 Kohlenstoffatomen im Arylteil und gegebenenfalls 1 bis 8 Kohlenstoffatomen im Alkylteil steht, wobei als Arylsubstituenten die oben aufgeführten Arylsubstituenten in Frage kommen oder für eine Gruppe -O-Z-NR^{10'}R^{11'}, -NR^{10'}R^{11'} oder -OM steht,
- R^{9'}: für Wasserstoff oder geradkettiges oder verzweigtes Alkyl mit 1 bis 6 Kohlenstoffatomen steht,
- R^{10'} und R^{11'}: gleich oder verschieden sind und für Wasserstoff, geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen stehen, wobei als Arylsubstituenten die oben aufgeführten Arylsubstituenten in Frage kommen,
- M: für Wasserstoff oder für ein Äquivalent eines entsprechenden Alkalimetall-, Erdalkalimetall- oder Ammoniumkations steht und
- Z: für eine geradkettige oder verzweigte Alkylkette mit 1 bis 8 Kohlenstoffatomen steht,
- A: für Wasserstoff oder eine Aminoschutzgruppe steht, und
- X und X¹: gleich oder verschieden sind und für Sauerstoff oder Schwefel stehen,
sowie deren Säureadditions-Salze und Metallsalz-Komplexe, ausgenommen die Verbindung 2-Hydroxymethyl-cyclohexyl-1-amin und Verbindungen in denen
a) R^{2'} für den Rest -CO-R^{8'} und A für H oder eine Aminoschutzgruppe und R^{1'}, R^{3'}, R^{4'}, R^{5'} und R^{6'} für H stehen, sowie
b) R^{2'} für den Rest -COOH und A und R^{1'} für H, und ein oder zwei R^{3'}, R^{4'}, R^{5'} und R^{6'} für C₁-C₄-Alkyl stehen.

Bevorzugte erfindungsgemäße Verbindungen sind auch Additionsprodukte aus Säuren und denjenigen substituierten 2-Cyclohexan-1-yl-amin-Derivaten der Formel (Ia) in denen R^{1'}, R^{2'}, R^{3'}, R^{4'}, R^{5'} und R^{6'} die oben angegebenen Bedeutungen haben.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Trifluoressigsäure, Maleinsäure, Bernsteinsäure, Fumarsäure, Weinsäure, Zitronensäure, Salicylsäure, Sorbinsäure und Milchsäure, Ölsäure, Stearinsäure, gegebenenfalls einfach oder mehrfach durch Nitro oder Halogen substituierte Benzoesäure, Gluconsäure, Ascorbinsäure, Äpfelsäure, Sulfamidsäure, Sulfonsäuren, wie z.B. p-Toluolsulfonsäure, 1,5-Naphthalindisulfonsäure und Methansulfonsäure, sowie Imide wie z.B. Phthalimid, Saccharin und Thiosaccharin.

Außerdem bevorzugte erfindungsgemäße Verbindungen sind auch Additionsprodukte aus Salzen von Metallen der I., II. und III. Hauptgruppen sowie des Zinns, sowie ferner Salze von Metallen der I., II., VII. und VIII. Nebengruppe des Periodensystems der Elemente und denjenigen substituierten 2-Cyclohexan-1-yl-amin-Derivaten der Formel (Ia), in denen R^{1'}, R^{2'}, R^{3'}, R^{4'}, R^{5'} und R^{6'} die oben angegebenen Bedeutungen haben.

Hierbei sind Salze des Kupfers, Zinks, Mangans, Magnesiums, Calciums, Zinns, Eisens, Cobalts und des Nickels besonders bevorzugt. Als Anionen dieser Salze kommen solche in Betracht, die sich von solchen Säuren ableiten, die zu physiologisch verträglichen Additionsprodukten führen, Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Besonders bevorzugt sind die Verbindungen der Formel (Ia), in welcher
- R^{1'}: für geradkettiges oder verzweigtes Alkyl mit 1 bis 4 Kohlenstoffatomen oder insbesondere für Wasserstoff steht,
- R^{2'}: für Formyl, geradkettiges oder verzweigtes Hydroxyalkyl mit 1 bis 4 Kohlenstoffatomen im Alkylteil oder für einen der Reste 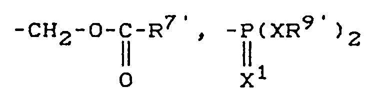 oder insbesondere 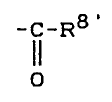 steht,
- R^{3'}, R^{4'}, R^{5'} und R^{6'}: gleich oder verschieden sind und jeweils für Wasserstoff, jeweils geradkettiges oder verzweigtes Alkyl oder Alkoxy mit 1 bis 6 Kohlenstoffatomen, Alkoxyalkyloxy mit jeweils 1 bis 6 Kohlenstoffatomen in den einzelnen Alkylteilen, für jeweils im Phenylteil unsubstituiertes oder einfach bis dreifach, gleich oder verschieden substituiertes Phenyl oder Phenylalkyl mit gegebenenfalls 1 oder 2 Kohlenstoffatomen im Alkylteil stehen, wobei als Phenylsubstituenten infrage kommen: Fluor, Chlor, Brom, Nitro, Cyano, Amino, C₁-C₂-Alkyl, C₁-C₃-Alkoxy, C₁-C₂-Alkylthio, Halogen-(C₁-C₂)-alkyl, Halogen-(C₁-C₂)-alkoxy, Halogen-(C₁-C₂)-alkylthio mit jeweils 1 bis 5 gleichen oder verschiedenen Fluor- und/ oder Chloratomen und Di-(C₁-C₂)-alkylamino,
weiterhin für eine unsubstituierte oder einfach bis dreifach, gleich oder verschieden substituierte und gegebenenfalls über eine Methylengruppe gebundene heterocyclische fünf-bzw, sechsgliedrige Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl stehen, wobei als Substituenten für den Heterocyclus jeweils infrage kommen: Fluor, Chlor, Brom, Nitro, Cyano, Amino, C₁-C₂-Alkyl, C₁-C₂-Alkoxy oder C₁-C₂-Alkylthio, Halogen-(C₁-C₂)-alkyl, Halogen-(C₁-C₂)-alkoxy, Halogen-(C₁-C₂)-alkylthio mit jeweils 1 bis 5 gleichen oder verschiedenen Fluor- und/oder Chloratomen und Di-(C₁-C₂)-alkylamino,
- insbesondere jedoch jeweils für Wasserstoff oder jeweils für geradkettiges oder verzweigtes Alkyl mit 1 bis 4 Kohlenstoffatomen stehen -
wobei mindestens zwei der Reste R^{3'}, R^{4'}, R^{5'} und R^{6'} für Wasserstoff stehen,
- R^{7'}: für jeweils geradkettiges oder verzweigtes Alkoxy oder insbesondere Alkyl mit 1 bis 4 Kohlenstoffatomen steht,
- R^{8'}: für Hydroxy, geradkettiges oder verzweigtes Hydroxyalkyloxy mit 1 bis 6 Kohlenstoffatomen, geradkettiges oder verzweigtes Halogenalkyloxy mit 1 bis 6 Kohlenstoffatomen und 1 bis 13 gleichen oder verschiedenen Halogenatomen, jeweils unsubstituiertes oder einfach bis dreifach, gleich oder verschieden durch Fluor, Chlor, Brom substituiertes Alkenylalkyloxy, Alkinylalkyloxy und Cycloalkyloxy mit 3 bis 6 Kohlenstoffatomen, jeweils geradkettiges oder verzweigtes Alkoxy oder Alkylthio mit 1 bis 4 Kohlenstoffatomen, geradkettiges oder verzweigtes Alkoxyalkylalkoxy mit jeweils 1 bis 4 Kohlenstoffatomen im Alkoxy- oder Alkylteil, jeweils im Phenylteil unsubstituiertes oder einfach bis dreifach, gleich oder verschieden substituiertes Phenyloxy, Phenylthio, Phenylalkyl oder Phenylalkyloxy mit jeweils gegebenenfalls 1 bis 6 Kohlenstoffatomen im Alkylteil steht, wobei als Phenylsubstituenten die oben aufgeführten Phenylsubstituenten in Frage kommen oder für eine Gruppe -O-Z-NR^{10'}R^{11'}, -NR^{10'}R^{11'} oder -OM steht,
- insbesondere jedoch für Hydroxy, jeweils geradkettiges oder verzweigtes Alkoxy, Alkoxyalkyloxy oder Phenylalkoxy mit jeweils 1 bis 4 Kohlenstoffatomen im Alkoxy- bzw. Alkylteil -
- R^{9'}: für Wasserstoff oder geradkettiges oder verzeigtes Alkyl mit 1 bis 4 Kohlenstoffatomen steht,
- R^{10'} und R^{11'}: gleich oder verschieden sind und für Wasserstoff, geradkettiges oder verzweigtes Alkyl mit 1 bis 4 Kohlenstoffatomen oder unsubstituiertes oder einfach bis dreifach, gleich oder verschieden substituiertes Phenyl stehen, wobei als Phenylsubstituenten die oben aufgeführten Phenylsubstituenten infrage kommen,
- M: für Wasserstoff oder für ein Äquivalent eines entsprechenden Alkalimetall-, Erdalkalimetall- oder Ammoniumkations steht und
- Z: für eine geradkettige oder verzweigte Alkylkette mit 1 bis 6 Kohlenstoffatomen steht,
- A: für Wasserstoff oder eine Aminoschutzgruppe steht und
- X und X¹: gleich oder verschieden sind und für Sauerstoff oder Schwefel stehen, ausgenommen Verbindungen in denen A, R^{3'}, R^{4'}, R^{5'} und R^{6'} für Wasserstoff, R^{2'} für Carboxy und R^{1'} für Methyl stehen.

In diesem Zusammenhang sind die gleichen Säureadditions-Salze und Metallsalz-Komplexe zu nennen, die bereits bei der Beschreibung der bevorzugten erfindungsgemäß substituierten 2-Cyclohexen-1-yl-amin-Derivate der Formel (Ia) genannt wurden.

Man erhält die substituierten 2-Cyclohexan-1-yl-amin-Derivate der Formel (Ia), wenn man
A) 2-Cyclohexan-1-yl-amin-Derivate der Formel (II) 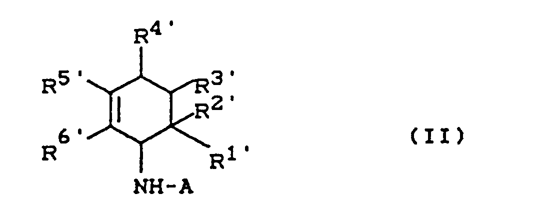 oder
B) Phenylamin-Derivate der Formel (III) 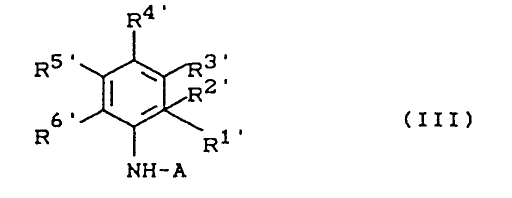 in welcher
   - R^{1'}, R^{2'}, R^{3'}, R^{4'}, R^{5'}, R^{6'} und A: jeweils die oben angegebene Bedeutung haben und
   in allgemein üblicher Weise, gegebenenfalls in Gegenwart eines Verdünnungsmittels, wie beispielsweise Ethanol, Dimethoxyethanol oder Tetrahydrofuran und in Gegenwart eines Katalysators, wie beispielsweise Ruthenium/ Kohlenstoff oder Rhodium auf Aluminiumoxid, bei Temperaturen von 10°C bis 300°C, und Drücken von 10 bis 300 bar mit Wasserstoff hydriert.
   Man erhält die substituierten 2-Cyclohexan-1-yl-amin-Derivate der Formel (Ia)
   in denen
   - A: für Wasserstoff steht, außerdem
C) aus den 2-Cyclohexan-Derivaten der Formel (Ia) 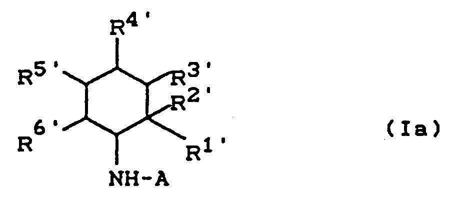 in welcher
   - R^{1'}, R^{2'}, R^{3'}, R^{4'}, R^{5'} und R^{6'}: die oben angegebene Bedeutung haben und
   - A: für eine Aminoschutzgruppe steht,
   in an sich bekannter Weise nach üblichen Methoden z.B. durch Solvolyse, wie Hydrolyse, Acidolyse, durch Reduktion, wie z.B. durch Hydrogenolyse in Gegenwart eines Hydrierungskatalysators oder mittels eines Reduktionssystems aus Metall und protonenabspaltendem Mittel, wobei je nach Art der Schutzgruppe verschiedenartige (auch andersartige) sowie selektive Abspaltungsmethoden angewendet werden können, gegebenenfalls in Gegenwart eines geeigneten Lösungs- oder Verdünnungsmittels oder eines Gemisches aus ihnen, wobei man je nach Bedarf unter Kühlen, bei Raumtemperatur oder unter Erwärmen, z.B. in einem Temperaturbereich von etwa -10°C bis zur Siedetemperatur des Reaktionsmediums, vorzugsweise von etwa -10°C bis etwa 150°C, und, falls erforderlich, in einem geschlossenen Gefäss, unter Druck, in einer Inertgasatmosphäre und/oder unter wasserfreien Bedingungen arbeitet
   und die so erhaltenen Produkte gegebenenfalls in Säureadditions-Salze oder Metallsalz-Komplexe überführt (vgl. Protective Groups in Organic Synthesis, Th. W. Greene, Wiley Interscience, 1981).
   Die vorne u.a. als Aminoschutzgruppe erwähnte Formyl-, Acetyl- oder 2,2,2-Trichloracetyl-gruppe kann beispielsweise durch Hydrolyse abgespalten werden.
   Die Hydrolyse erfolgt in an sich bekannter Weise mit Hilfe von Wasser, wobei vorteilhaft in Gegenwart einer die Hydrolyse unterstützenden Säure oder Base, gegebenenfalls in Gegenwart eines inerten Lösungsmittels oder Verdünnungsmittels und/oder unter Kühlen oder Erwärmen gearbeitet wird.
   Als Säuren kommen beispielsweise anorganische Säuren, wie Mineralsäuren, z.B. Schwefelsäure, Phosphorsäure oder Halogenwasserstoffsäuren, organische Carbonsäuren, wie Niederalkancarbonsäuren, z.B. Eisessig, wie gegebenenfalls ungesättigte Dicarbonsäuren, z.B. Oxal-, Malon-, Malein- oder Fumarsäure, oder wie Hydroxycarbonsäuren, z.B. Weinsäure oder Citronensäure, oder Sulfonsäuren, wie C₁-C₇-Alkan- oder gegebenenfalls substituierte Benzolsulfonsäure, z.B. Methan- oder p-Toluolsulfonsäure, in Betracht.
   Als Basen kommen beispielsweise Alkalimetallhydroxide, -hydride, -amide, -alkanolate, -carbonate, -triphenylmethylide, -di-C₁-C₇-alkylamide, -amino-C₁-C₇-alkylamide oder -C₁-C₇-alkylsilylamide, Naphthalin-amine, C₁-C₇-Alkylamine, basische Heterocyclen, Ammoniumhydroxide sowie carbocyclische Amine in Frage. Beispielhaft seien Lithium-hydroxid, Natrium-hydroxid, -hydrid, -amid, -ethylat, Kalium-tert-butylat, -carbonat, Lithiumtriphenylmethylid, -diisopropylamid, Kalium-3-(aminopropyl)amid, -bis-(trimethylsilyl)-amid, Dimethyl-aminonaphthalin, Di- oder Triethylamin, Pyridin, Benzyltrimethyl-ammoniumhydroxid, 1,5-Diazabicyclo-[4.3.0]non-5-en (DBN) sowie 1,8-Diaza-bicyclo-[5.4.0]undec-7-en (DBU) genannt.
   Die Acidolyse gelingt z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure, Die Anwesenheit eines zusätzlichen inerten Lösungsmittels ist möglich. Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser.
   Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure vorzugsweise in Form eines Gemisches aus Essigsäure und 70 %iger Perchlorsäure im Verhältnis 9 : 1. Die Reaktionstemperaturen für diese Solvolysen liegen zweckmäßig zwischen etwa 0 und etwa 50°C, vorzugsweise arbeitet man zwischen 15 und 30°C (Raumtemperatur).
   Die BOC-Gruppe kann z.B. bevorzugt mit 40 %iger Trifluoressigsäure in Methylenchlorid oder mit etwa 3 bis 5 n Salzsäure in Dioxan bei 15 - 30°C abgespalten werden, die FMOC-Gruppe (9-Fluorenylmethyloxycarbonyl) mit einer etwa 5-bis 20%igen Lösung von Dimethylamin, Diethylamin oder Piperidin in Dimethylformamid bei 15 - 30°C. Eine Abspaltung der DNP-Gruppe (2,4-Dinitrophenyl) gelingt z.B. auch mit einer etwa 3- bis 10%igen Lösung von 2-Mercaptoethanol in Dimethylformamid/Wasser bei 15-30°C. Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden, Als Lösungsmittel eignen sich dabei die oben angegebenen, insbesondere z.B. Alkohole, wie Methanol oder Ethanol oder Amide wie Dimethylformamid. Die Hydrogenolyse wird in der Regel bei Temperaturen von etwa 0 bis 100°C und einem Druck von etwa 1 bis 200 bar, bevorzugt bei 20 bis 30°C und 1 bis 10 bar durchgeführt. Eine Hydrogenolyse der CBZ-Gruppe gelingt z.B. gut an 5- bis 10%igem Pd-Kohle in Methanol bei 20 -30°C.

Als Aminoschutzgruppen, die mittels eines Reduktionssystems aus Metall und protonenabspaltendem Mittel abgespalten werden, sind beispielsweise (4-Nitro)-benzyloxycarbonyl, 2-Iod- oder 2,2,2-Trichlorethoxycarbonyl oder Phenacyloxycarbonyl, zu nennen.

Der Metallbestandteil des metallischen Reduktionssystems ist beispielsweise ein unedles Metall, wie Alkali- oder Erdalkalimetall, z.B. Lithium, Natrium, Kalium, Magnesium oder Calcium, oder Übergangsmetall, z.B. Zink, Zinn, Eisen oder Titan, während als Protonen-abspaltendes Mittel, z.B. Protonsäuren der vorstehend genannten Art, wie Salz- oder Essigsäure, C₁-C₇-Alkohole, wie Ethanol, und/oder Amine bzw. Ammoniak in Frage kommen, Solche Systeme sind beispielsweise Natrium/Ammoniak, Zink/Salz-oder Essigsäure oder Zink/Ethanol.

4-Nitrobenzyloxycarbonyl kann ferner z.B. mit einem Dithionit, wie Natriumdithionit, Phenacyloxycarbonyl und 2-Halogen-C₂-C₇-alkanoyl z.B. mit Hilfe eines nucleophilen Reagens, wie einem Thiolat, z.B. Natriumthiophenolat, oder Thioharnstoff und Base und sich anschließender Hydrolyse, und Allyl oder But-2-enyl, mit Hilfe eines Rhodium(III)halogenids, wie Rhodium(III) chlorid, gespalten werden.

Die bekannten Verbindungen der Formel (Ia) lassen sich in Analogie zu den neuen Verbindungen der Formel (Ia) herstellen.

Verwendet man als Ausgangsstoffe beispielsweise tert.-Butyl-(6-carbomethoxy-4-methyl-2-cyclohexen-1-yl)-carbonat, so läßt sich der Reaktionsablauf des Herstellungsverfahrens (A) durch das folgende Formelschema darstellen:

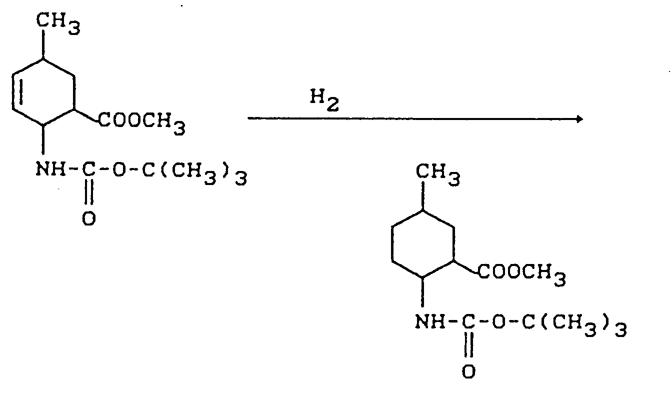

Verwendet man als Ausgangsstoffe beispielsweise tert-Butyl-(4-methyl-2-carbomethoxy-cyclohexan-1-yl)-carbamat und 1 N Natronlauge in einer ersten Stufe und 1 N Salzsäure in einer zweiten Stufe, so läßt sich der Reaktionsablauf des Herstellungsverfahrens (C) durch das folgende Formelschema darstellen:

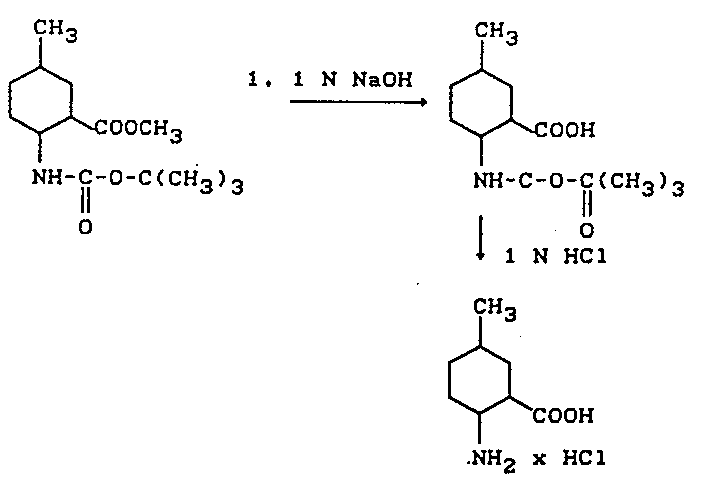

Die zur Durchführung des Herstellungsverfahrens (A) als Ausgangsstoffe benötigten 2-Cyclohexen-1-yl-amin-Derivate bzw. die Phenylamin-Derivate sind durch die Formel (II) bzw. Formel (III) allgemein definier. In dieser Formel (II) bzw. Formel (III) stehen R^{2'}, R^{3'}, R^{4'}, R^{5'} und R^{6'} und R^{1'} in Formel (II) vorzugsweise bzw. insbesondere für diejenigen Substituenten, die oben bei der Beschreibung der neuen 2-Cyclohexan-1-yl-amin-Derivate der Formel (Ia) als bevorzugt bzw. besonders bevorzugt für diese Reste genannt wurden.

Die 2-Cyclohexen-1-yl-amin-Derivate der Formel (II) und die Phenylamin-Derivate der Formel (III) sind bekannt und können nach bekannten Verfahren in einfacher analoger Weise hergestellt werden.

Die erfindungsgemäßen Verfahren A und B zur Herstellung der neuen Cyclohexan-Derivate der Formel (Ia) werden vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, sowie Dimethylsulfoxid, Tetramethylensulfon und Hexamethylphosphorsäuretriamid.

Als Inertgase kommen dabei Stickstoff sowie praktisch alle Edelgase, insbesondere Argon infrage.

Die Reaktionstemperaturen können bei den Verfahren A und B zur Herstellung der 2-Cyclohexan-Derivate der Formel (Ia) in einem größeren Bereich variiert werden. Im allgemeinen arbeitet man bei Temperaturen zwischen 10°C und 300°C vorzugsweise zwischen 20°C und 150°C.

Zur Durchführung der Verfahren A und B zur Herstellung der 2-Cyclohexan-Derivate der Formel (Ia) setzt man Wasserstoff im Überschuß und vorzugsweise 0,1 bis 5,0 Mol an Katalysator ein.

Das erfindungsgemäße Verfahren zur Herstellung der 2-Cyclohexan-Derivate der Formel (Ia) wird im allgemeinen unter erhöhtem Druck durchgeführt. Im allgemeinen arbeitet man bei einem Druck von 1 bis 300 bar, vorzugsweise bei 5 bis 200 bar.

Für die Herstellung der neuen 2-Cyclohexan-1-yl-carbonsäure-Derivate der Formel (Ia) gemäß Verfahrensvarianten A und B kommen die für derartige Reaktionen üblichen Katalysatoren infrage; vorzugsweise verwendet man Edelmetallkatalysatoren wie beispielsweise. Ruthium auf Kohlenstoff oder Rhodium auf Aluminiumoxid.

Das Verfahren zur Herstellung der neuen 2-Cyclohexan-Derivate der Formel (Ia) kann unter bestimmten Voraussetzungen jedoch auch ohne Verdünnungsmittel und einem Druck von 1 bis 200 bar durchgeführt werden.

Die Reaktionen werden im allgemeinen in einem geeigneten Verdünnungsmittel durchgeführt und das Reaktionsgemisch wird mehrere Stunden bei der jeweils erforderlichen Temperatur gerührt. Die Aufarbeitung erfolgt jeweils nach üblichen Methoden. Im allgemeinen verfährt man in der Weise, daß man das Reaktionsgemisch entweder unter vermindertem Druck einengt oder in Wasser gießt, das Produkt durch Extraktion oder Filtration isoliert und durch Chromatographie reinigt.

Die Verbindungen der Formel (Ia) können als Enantiomeren- oder Diastereomerengemische anfallen.

Die Erfindung umfaßt sowohl die reinen Isomeren als auch die Gemische. Diese Gemische von Diastereomeren können nach gebräuchlichen Methoden, zum Beispiel durch selektive Kristallisation, aus geeigneten Lösungsmitteln oder Chromatographie an Kieselgel oder Aluminiumoxid in die Komponenten aufgetrennt werden. Racemate können nach üblichen Methoden in die einzelnen Enantiomeren aufgetrennt werden, so zum Beispiel durch Salzbildung mit optisch aktiven Säuren wie Camphersulfonsäure oder Dibenzoylweinsäure und selektive Kristallisation, oder durch Derivatisierung mit geeigneten optisch aktiven Reagenzien, Trennung der diastereomeren Derivate und Rückspaltung oder Trennung an optisch aktivem Säulenmaterial.

Zur Herstellung von Säureadditions-Salzen der Verbindungen der Formel (Ia) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (Ia) können in einfacher Weise nach üblichen Salzbildungsmethoden, z.B. durch Lösen einer Verbindung der allgemeinen Formel (Ia) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (Ia) kommen vorzugsweise diejenigen Salze von Metallen infrage, die bereits weiter oben beschrieben wurden.

Die Metallsalz-Komplexe von Verbindungen der allgemeinen Formel (Ia) können in einfacher Weise nach üblichen Verfahren erhalten werden, so z.B. durch Lösen des Metallsalzes in Alkohol, z.B. Ethanol, und Hinzufügen zu Verbindungen der allgemeinen Formel (Ia). Man kann Metallsalz-Komplexe in bekannter Weise, z.B. durch Abfiltrieren, isolieren und gegebenenfalls durch Umkristallisation reinigen.

Die erfindungsgemäß verwendbaren Wirkstoffe der Formel (Ia) und ihre Säureadditionssalze weisen eine starke biologische Wirkung auf und können zur Bekämpfung von unerwünschten Schädlingen praktisch eingesetzt werden. Die Wirkstoffe sind z. B. für den Gebrauch als Pflanzenschutzmittel einsetzbar, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform; Drechslera, Syn: Helminthosporium);
Cochliobolus-Arten, wie beispielsweise Cochliobolus sativus (Konidienform; Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäß verwendbaren Wirkstoffe mit besonders gutem Erfolg protektiv zur Bekämpfung von Phytophthora-Arten an Tomaten und Venturia-Arten bei Äpfeln eingesetzt werden.

Außerdem besitzen einige der erfindungsgemäß verwendbaren Wirkstoffe gegen Pythium-Arten, Alternaria-Arten und Cercospora-Arten eine gute Wirkung.

Die erfindungsgemäß verwendbaren Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage; Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage; z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage; z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage; z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.
Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäß verwendbaren Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäß verwendbaren Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die erfindungsgemäß verwendbaren Verbindungen der Formel (Ia) und ihre Säureadditions-Salze weisen desweiteren antimikrobielle, insbesondere starke antibakterielle und antimykotische Wirkungen auf. Sie besitzen ein sehr breites antimykotisches Wirkungsspektrum, insbesondere gegen Dermatophyten und Sproßpilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Die Herstellung und die Verwendung der erfindungsgemäßen Wirkstoffe geht aus den nachfolgenden Beispielen hervor.

### Herstellungsbeispiele

### Beispiel 1 (Verfahren A)

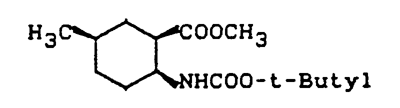

### cis-tert.-Butyl-2-carbomethoxy-4-methyl-cyclohexan-1-yl)-carbamat

Eine Lösung von 30 g (0,11 Mol) cis-tert.-Butyl-(4-methyl-6-carbomethoxy-2-cyclohexen-1-yl)-carbamat in 100 ml Ethanol wird bei Raumtemperatur und 30 bar H₂ bis zur Beendigung der Wasserstoffaufnahme hydriert. Man filtriert vom Katalysator ab, engt das Filtrat bis zur Trockne ein, und erhält 28 g (93 % d. Theorie) cis-tert.-Butyl-(2-carbomethoxy-4-methyl-cyclohexan-1-yl)-carbamat mit dem Schmelzpunkt von 74-79° C.

### Beispiel 2

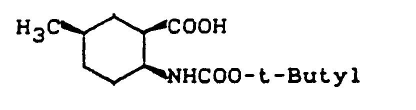

### cis-tert.-Butyl-(2-carboxy-4-methyl-cyclohexan-1-yl)-carbamat

15 g (0,055 Mol) fein gemörsertes cis-tert.-Butyl-(2-carbomethoxy-4-methyl-2-cyclohexen-1-yl)-carbamat werden in 60 ml 1N Natronlauge suspendiert und 20 h bei 50° C gerührt. Nach dem Abkühlen extrahiert man einmal mit Diethylether, stellt die wässrige Phase mit konz, Salzsäure bei 0°C auf pH 1 ein, und saugt den Feststoff ab. Nach Waschen mit Wasser und Trocknen erhält man 13,3 g (94 % d. Theorie) cis-tert.-Butyl-(2-carboxy-4-methylcyclohexan-1-yl)-carbamat mit dem Schmelzpunkt von 184-188° C.

### Beispiel 3 (Verfahren C)

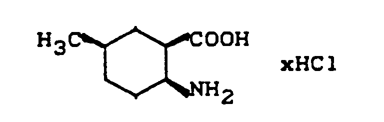

### cis-2-carboxy-4-methylcyclohexan-1-yl-amin-hydrochlorid

5 g (0,02 Mol) cis-tert.-Butyl-(2-carboxy-4-methylcyclohexan-1-yl)-carbamat werden in 20 ml 1N Salzsäure suspendiert und 12 h bei 55 bis 60° C gerührt. Nach dem Einengen und Trocknen im Hochvakuum werden 3,7 g (96 % d.Theorie) cis-2-carboxy-4-methylcyclohexan-1-yl-aminhydrochlorid als weißer Feststoff mit dem Schmelzpunkt von 218-230° C erhalten.

### Beispiel 4 (Verfahren B)

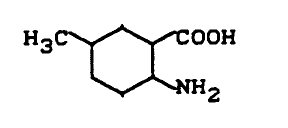

### 2-Carboxy-4-methyl-cyclohexan-1-yl-amin

50 g (0,33 Mol) 2-Amino-5-methyl-benzoesäure, in 100 ml Tetrahydrofuran gelöst, werden in Gegenwart von 5 g Ruthenium/Kohlenstoff 14 h bei 200°C und 200 bar Wasserstoff hydriert. Nach dem Abkühlen und Einengen werden 30 g 2-Carboxy-4-methyl-cyclohexan-1-yl-amin als helles Öl erhalten.
¹H-NMR (200 MHz, CDCl₃):
δ = 0,85-0,98 (m, 3H); 1,05-2,00(m, 9H); 2,35-2,68 (m, 1H).

### Beispiel 5

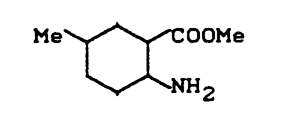

### 2-Carboxymethyl-4-methyl-cyclohexan-1-yl-amin

Unter N₂ fügt man bei -5°C 7,3 ml (0,1 Mol) Thionylchlorid zu 30 ml Methanol, und tropft dann zu dieser Lösung 15 g (0,096 Mol) 2-Carboxy-4-methyl-cyclohexan-1-yl-amin in 10 ml Methanol bei 0°C zu. Nach 12-stündigem Rühren unter Rückfluß kühlt man ab, fügt weitere 10 ml Methanol und 3 ml (0,04 Mol) Thionylchlorid zu, und rührt weitere 4 Stunden unter Rückfluß. Nach Abkühlen und Einengen zur Trockne werden 5 g des Rückstandes in ca. 50 ml Diethylether aufgenommen und ca. 100 ml kalter 1N Natonlauge und Wasser gewaschen. Die organische Phase wird getrocknet und eingeengt. Man erhält 3,5 g (85 % d.Theorie) 2-Carboxpethyl-4-methyl-cyclohexan-1-yl-amin als hellgelbes Öl.
¹H-NMR (200 MHz, CDCl₃);
δ = 3,70 (s, 3H, -CO₂CH₃).

In analoger Weise zu den in den Beispiel 1 bis 5 beschriebenen Methoden und unter Berücksichtigung der Angaben in der Beschreibung zu den erfindungsgemäßen Verfahren werden die in der nachfolgenden Tabelle 1 aufgeführten Endprodukte der Formel (Ia) 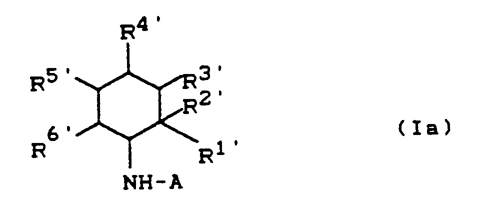 erhalten.

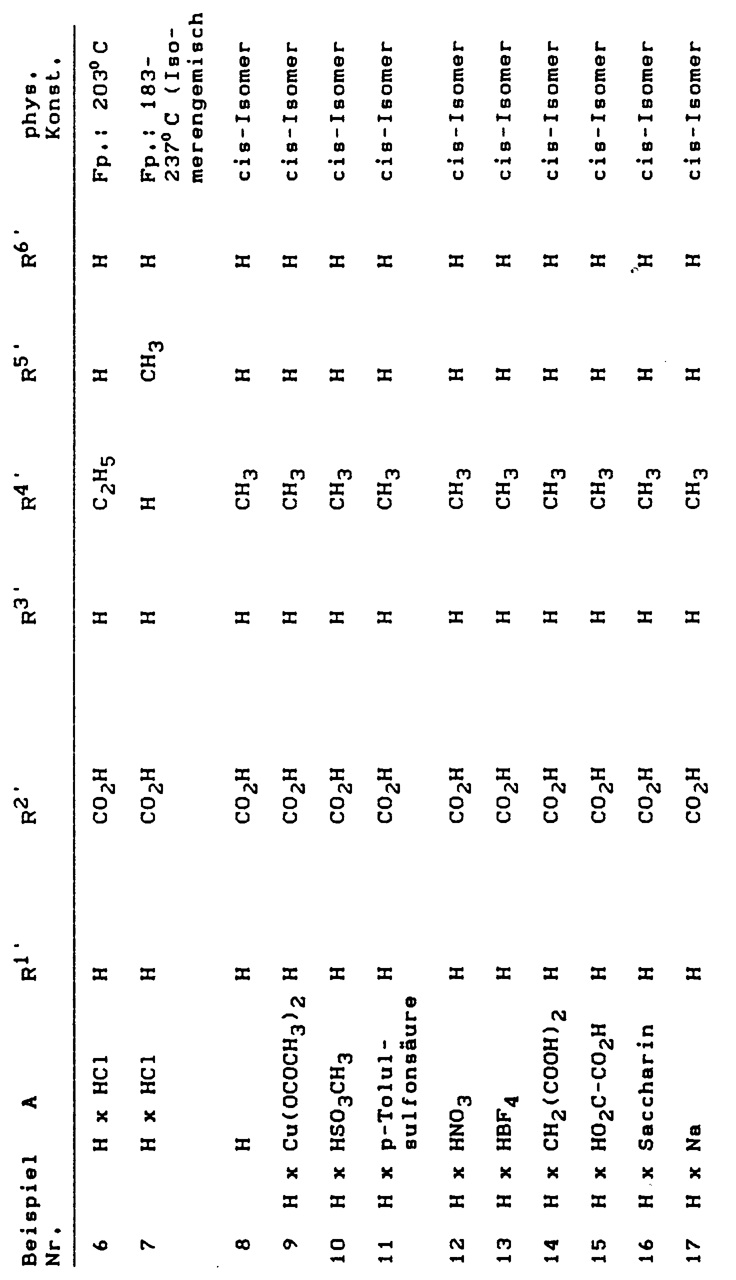

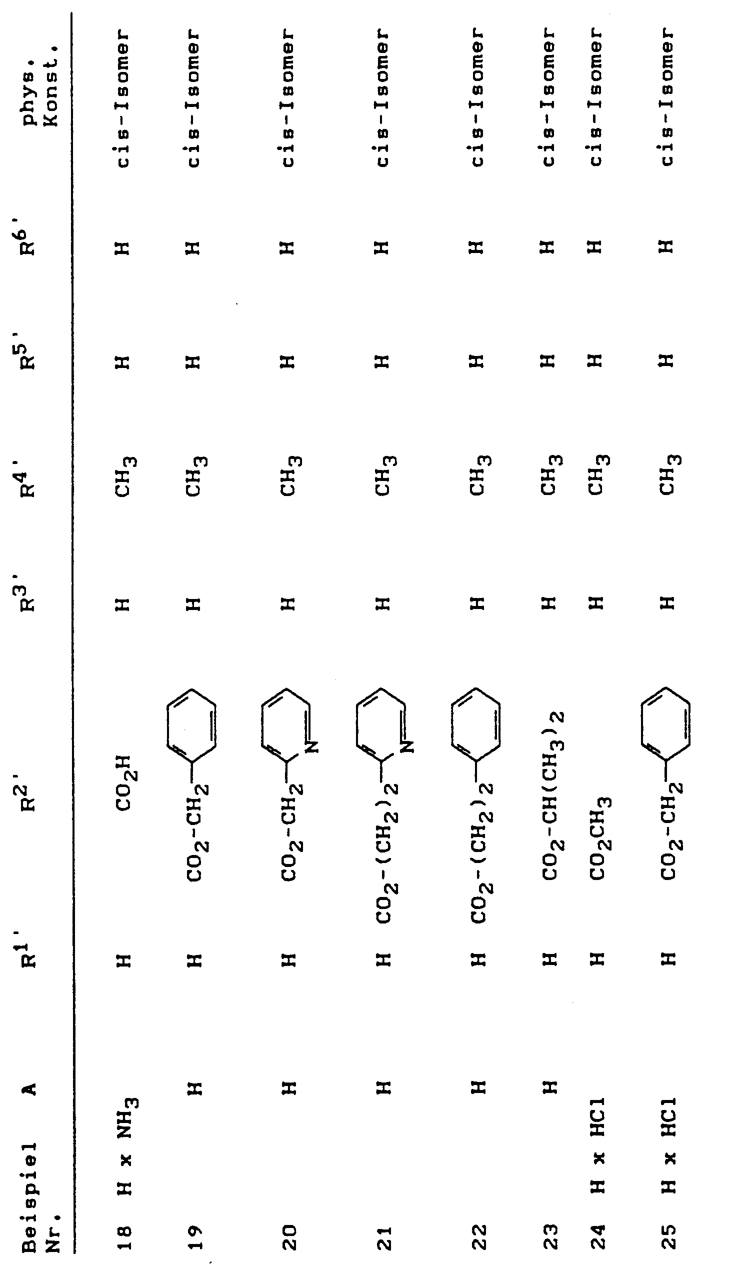

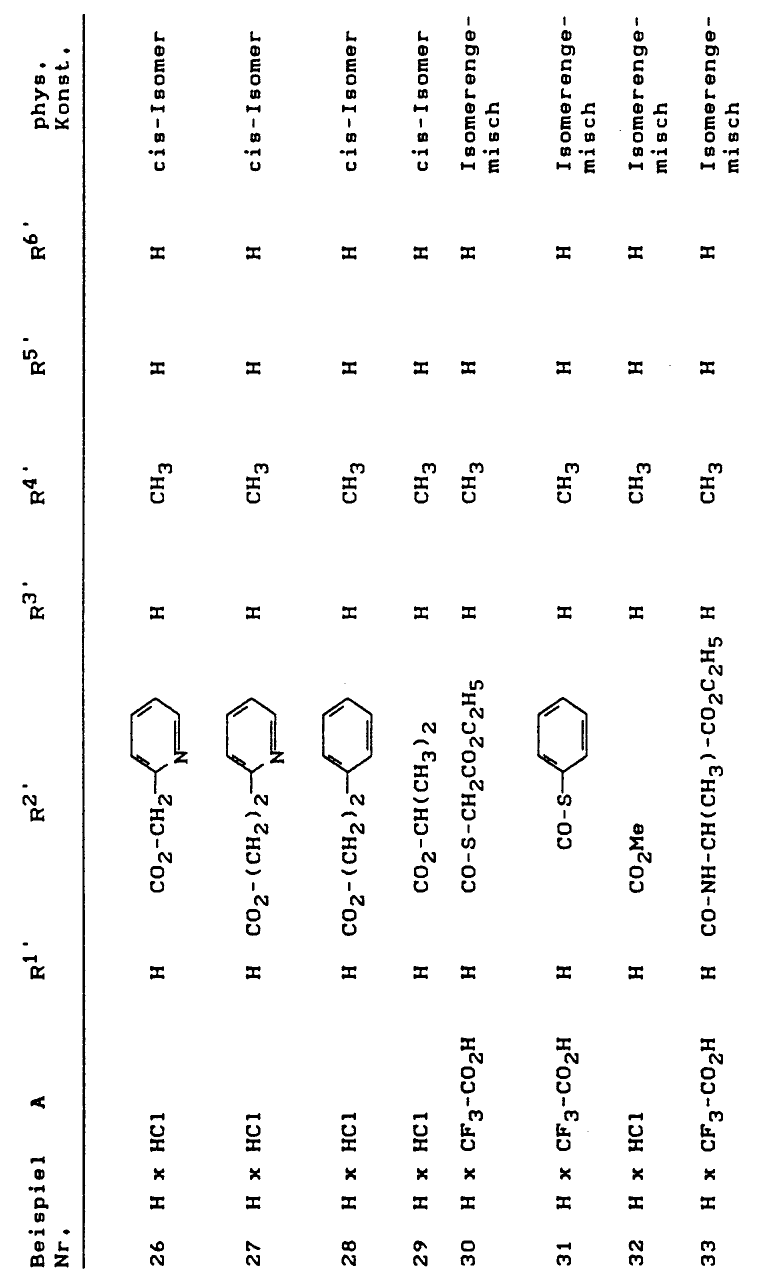

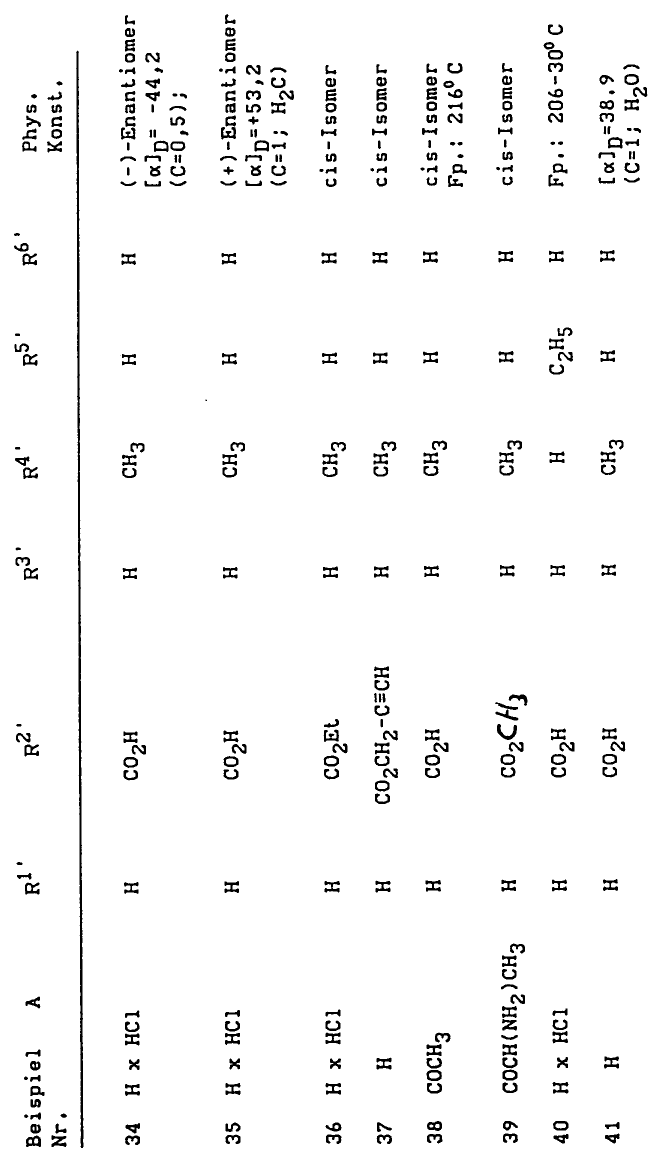

### Herstellung der Ausgangsverbindungen

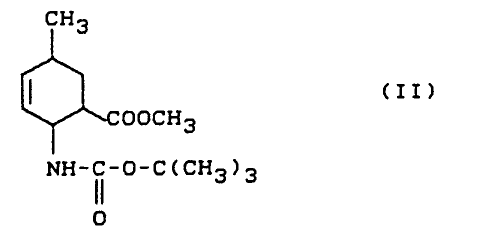

Eine Lösung aus 10 g (0,05 Mol) 2-Carboxy-5-methylcyclohex-3-en-carbonsäuremethylester und 8 g (0,062 Mol) N,N-Diisopropylethylamin in 30 ml Aceton wird bei -5° C mit einer Lösung aus 5,4 g (0,05 Mol) Chlorameisensäureethylester in 15 ml Aceton über 30 Minuten versetzt. Nach weiteren 30 Minuten bei 0°C tropft man eine eisgekühlte Lösung aus 6,5 g (0,1 Mol) Natriumazid in 15 ml Wasser zu. Man läßt 15 Minuten bei 0°C rühren und arbeitet dann mit Wasser-Toluol auf.

Die auf ca. 50 ml eingeengte Toluol-Phase wird dann zu einer unter Rückfluß siedenden Lösung aus 3 g (0,04 Mol) tert-Butanol, 25 mg (0,15 mMol) tert-Butylcatechol in 20 ml Toluol getropft. Der Reaktionsverlauf wird IR-spektroskopisch verfolgt.

Man läßt auf Raumtemperatur abkühlen und engt ein. Nach säulenchromatographischer Trennung an Kieselgel mit dem Laufmittel Petrolether-Essigsäureethylester (6 : 1) erhält man 4 g (30 % der Theorie) tert-Butyl-(4-methyl-6-carbomethoxy-2-cyclohexen-1-yl)-carbamat vom Schmelzpunkt 89-91° C.

### Beispiel A

### Phytophthora-Test (Tomate) /protektiv

- Lösungsmittel:: 4,7 Gewichtsteile Aceton
- Emulgator:: 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mmit einer wäßrigen Sporensuspension von Phytophthora infestans inokuliert.

Die Pflanzen werden in einer Inkubationskabine mit 100% relativer Luftfeuchtigkeit und ca. 20° C aufgestellt.

3 Tage nach der Inokulation erfolgt die Auswertung.

Der Wirkungsgrad der Verbindungen 3, 8, 9, 10, 11, 12, 13, 14, 15, 16, 17 und 18 liegt bei einer Wirkstoffkonzentration von 10 ppm über 90%.

### Beispiel B

### Pythium sp.-Test (Erbse) / Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glasflasche.

Das Saatgut sät man mit 2 x 50 Korn 2 cm tief in eine mit Pythium sp. natürlich infizierte Komposterde und kultiviert es im Gewächshaus bei einer Temperatur von ca. 20° C in Saatkästen, die täglich 15 Stunden dem Licht ausgesetzt werden.

Nach 14 Tagen erfolgt die Auswertung.

Die Verbindung 3 zeigt bei einer Wirkstoffaufwandmenge von 250 mg/kg Saatgut eine ausgezeichnete Wirkung.

## Patentansprüche

1. Verwendung der Verbindungen der Formel (Ia) 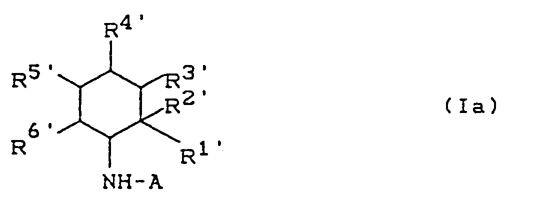 in welcher
R^{1'} für Wasserstoff, Halogen oder geradkettiges oder verzweigtes Alkyl mit 1 bis 6 Kohlenstoffatomen steht,
R^{2'} für Formyl, geradkettiges oder verzweigtes Hydroxyalkyl mit 1 bis 8 Kohlenstoffatomen im Alkylteil oder für einen der Reste 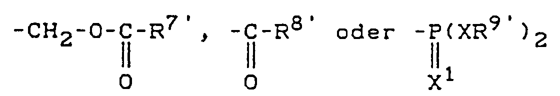 steht,
R^{3'}, R^{4'}, R^{5'} und R^{6'} gleich oder verschieden sind und jeweils für Wasserstoff, jeweils geradkettiges oder verzweigtes Alkyl oder Alkoxy mit 1 bis 8 Kohlenstoffatomen, Alkoxyalkyloxy mit jeweils 1 bis 8 Kohlenstoffatomen in den einzelnen Alkylteilen, für jeweils im Arylteil unsubtituiertes oder einfach bis fünffach, gleich oder verschieden substituiertes Aryl oder Aralkyl mit jeweils 6 bis 10 Kohlenstoffatomen im Arylteil und gegebenenfalls 1 bis 4 Kohlenstoffatomen im Alkylteil stehen, wobei als Arylsubstituenten infrage kommen: Halogen, Nitro, Cyano, Amino, C₁-C₄-Alkyl, C₁-C₄-Alkoxy, C₁-C₄-Alkylthio, Halogen-(C₁-C₄)-alkyl, Halogen-(C₁-C₄)-alkoxy, Halogen-(C₁-C₄)-alkylthio mit jeweils 1 bis 9 gleichen oder verschiedenen Halogenatomen und Di-(C₁-C₄)-alkylamino,
weiterhin für eine unsubstituierte oder einfach bis dreifach, gleich oder verschieden substituierte und gegebenenfalls über eine Methylengruppe gebundene heterocyclische 5- oder 6-gliedrige Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl stehen, wobei als Substituen für den Heteroyclus jeweils infrage kommen: Halogen, Nitro, Cyano, Amino, C₁-C₄-Alkyl, C₁-C₄-Alkoxy oder C₁-C₄-Alkylthio, Halogen-(C₁-C₄-alkyl, Halogen-(C₁-C₄)-alkoxy, Halogen-(C₁-C₄)-alkylthio mit jeweils 1 bis 9 gleichen oder verschiedenen Halogenatomen und Di-(C₁-C₄)-alkylamino,
wobei mindestens zwei der Reste R^{3'}, R^{4'}, R^{5'} oder R^{6'} für Wasserstoff stehen.
R^{7'} für jeweils geradkettiges oder verzweigtes Alkyl der Alkoxy mit 1 bis 6 Kahlenstoffatomen steht,
R^{8'} für Hydroxy, geradkettiges oder verzweigtes Hydroxyalkyloxy mit 1 bis 8 Kohlenstoffatomen,
geradkettiges oder verzweigtes Halogenalkyloxy mit 1 bis 8 Kohlenstoffatomen und 1 bis 17 gleichen oder verschiedenen Halogenatomen, jeweils unsubstituiertes oder einfach bis mehrfach, gleich oder verschieden durch Halogen substituiertes Alkenylalkyloxy, Alkinylalkyloxy und Cycloalkyloxy mit 3 bis 6 Kohlenstoffatomen, jeweils geradkettiges oder verzweigtes Alkoxy oder Alkylthio mit 1 bis 6 Kohlenstoffatomen, geradkettiges oder verzweigtes Alkoxyalkyloxy mit jeweils 1 bis 6 Kohlenstoffatomen im Alkoxy- oder Alkylteil, jeweils im Arylteil unsubstituiertes oder einfach bis fünffach, gleich oder verschieden substituiertes Aryloxy, Arylthio, Aralkyl oder Aralkyloxy mit jeweils 6 bis 10 Kohlenstoffatomen im Arylteil und gegebenenfalls 1 bis 8 Kohlenstoffatomen im Alkylteil steht, wobei als Arylsubstituenten die oben aufgeführten Arylsubstituenten in Frage kommen oder für eine1Gruppe -O-Z-NR^{10'}R^{11'},
-NR^{10'}R^{11'} oder -OM steht,
R^{9'} für Wasserstoff oder geradkettiges oder verzweigtes Alkyl mit 1 bis 6 Kohlenstoffatomen steht,
R^{10'} und R^{11'} gleich oder verschieden sind und für Wasserstoff, geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen stehen,
wobei als Arylsubstituenten die oben aufgeführten Arylsubstituenten in Frage kommen,
M für Wasserstoff oder für ein Äquivalent eines entsprechenden Alkalimetall-, Erdalkalimetall- oder Ammoniumkations steht und
Z für eine geradkettige oder verzweigte Alkylkette mit 1 bis 8 Kohlenstoffatomen steht
A für Wasserstoff oder eine Aminoschutzgruppe steht und
X und X¹ gleich oder verschieden sind und für Sauerstofff oder Schwefel stehen,
sowie deren Säureadditions-Salze und Metallsalz-Komplexe,
als Schädlingsbekämpfungsmittel.

2. Substituierte Cyclohexan-1-yl-amin-Derivate der Formel (Ia) 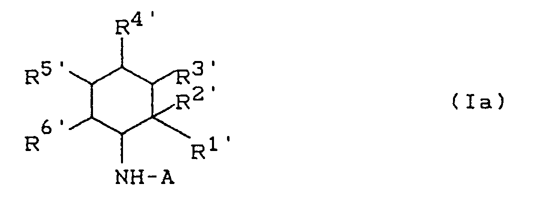 in welcher
R^{1'} für Wasserstoff, Halogen oder geradkettiges oder verzweigtes Alkyl mit 1 bis 6 Kohlenstoffatomen steht,
R^{2'} für Formyl, geradkettiges oder verzweigtes Hydroxyalkyl mit 1 bis 8 Kohlenstoffatomen im Alkylteil oder für einen der Reste 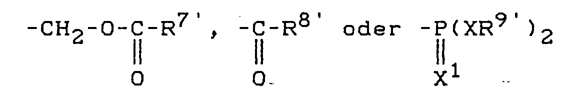 steht,
R^{3'}, R^{4'}, R^{5'} und R^{6'} gleich oder verschieden sind und jeweils für Wasserstoff, jeweils geradkettiges oder verzweigtes Alkyl oder Alkoxy mit 1 bis 8 Kohlenstoffatomen, Alkoxyalkyloxy mit jeweils 1 bis 8 Kohlenstoffatomen in den einzelnen Alkylteilen, für jeweils im Arylteil unsubtituiertes oder einfach bis fünffach, gleich oder verschieden substituiertes Aryl oder Aralkyl mit jeweils 6 bis 10 Kohlenstoffatomen im Arylteil und gegebenenfalls 1 bis 4 Kohlenstoffatomen im Alkylteil stehen, wobei als Arylsubstituenten infrage kommen: Halogen, Nitro, Cyano, Amino, C₁-C₄-Alkyl, C₁-C₄-Alkoxy, C₁-C₄-Alkylthio, Halogen-(C₁-C₄)-alkyl, Halogen-(C₁-C₄)-alkoxy, Halogen-(C₁-C₄)-alkylthio mit jeweils 1 bis 9 gleichen oder verschiedenen Halogenatomen und Di-(C₁-C₄)-alkylamino,
weiterhin für eine unsubstituierte oder einfach bis dreifach, gleich oder verschieden substituierte und gegebenenfalls über eine Methylengruppe gebundene heterocyclische 5- oder 6-gliedrige Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl stehen, wobei als Substituen für den Heteroyclus jeweils infrage kommen: Halogen, Nitro, Cyano, Amino, C₁-C₄-Alkyl, C₁-C₄-Alkoxy oder C₁-C₄-Alkylthio, Halogen-(C₁-C₄-alkyl, Halogen-(C₁-C₄)-alkoxy, Halogen-(C₁-C₄)-alkylthio mit jeweils 1 bis 9 gleichen oder verschiedenen Halogenatomen und Di-(C₁-C₄)-alkylamino,
wobei mindestens zwei der Reste R^{3'}, R^{4'}, R^{5'} oder R^{6'} für Wasserstoff stehen.
R^{7'} für jeweils geradkettiges oder verzweigtes Alkyl der Alkoxy mit 1 bis 6 Kohlenstoffatomen steht,
R^{8'} für Hydroxy, geradkettiges oder verzweigtes Hydroxyalkyloxy mit 1 bis 8 Kohlenstoffatomen,
geradkettiges oder verzweigtes Halogenalkyloxy mit 1 bis 8 Kohlenstoffatomen und 1 bis 17 gleichen oder verschiedenen Halogenatomen, jeweils unsubstituiertes oder einfach bis mehrfach, gleich oder verschieden durch Halogen substituiertes Alkenylalkyloxy, Alkinylalkyloxy und Cycloalkyloxy mit 3 bis 6 Kohlenstoffatomen, jeweils geradkettiges oder verzweigtes Alkoxy oder Alkylthio mit 1 bis 6 Kohlenstoffatomen, geradkettiges oder verzweigtes Alkoxyalkyloxy mit jeweils 1 bis 6 Kohlenstoffatomen im Alkoxy- oder Alkylteil, jeweils im Arylteil unsubstituiertes oder einfach bis fünffach, gleich oder verschieden substituiertes Aryloxy, Arylthio, Aralkyl oder Aralkyloxy mit jeweils 6 bis 10 Kohlenstoffatomen im Arylteil und gegebenenfalls 1 bis 8 Kohlenstoffatomen im Alkylteil steht, wobei als Arylsubstituenten die oben aufgeführten Arylsubstituenten in Frage kommen oder für eine1Gruppe -O-Z-NR^{10'}R^{11'},
-NR^{10'}R^{11'} oder -OM steht,
R^{9'} für Wasserstoff oder geradkettiges oder verzweigtes Alkyl mit 1 bis 6 Kohlenstoffatomen steht,
R^{10'} und R^{11'} gleich oder verschieden sind und für Wasserstoff, geradkettiges oder verzweigtes Alkyl mit 1 bis 6 Kohlenstoffatomen oder unsubstituiertes oder einfach bis fünffach, gleich oder verschieden substituiertes Aryl mit 6 bis 10 Kohlenstoffatomen stehen,
wobei als Arylsubstituenten die oben aufgeführten Arylsubstituenten in Frage kommen,
M für Wasserstoff oder für ein Äquivalent eines entsprechenden Alkalimetall-, Erdalkalimetall- oder Ammoniumkations steht und
Z für eine geradkettige oder verzweigte Alkylkette mit 1 bis 8 Kohlenstoffatomen steht
A für Wasserstoff oder eine Aminoschutzgruppe steht und
X und X¹ gleich oder verschieden sind und für Sauerstofff oder Schwefel stehen,
sowie deren Säureadditions-Salze und Metallsalz-Komplexe, ausgenommen die Verbindung 2-Hydroxymethyl-cyclohexyl-1-amin und Verbindungen in denen
a) R^{2'} für den Rest -CO-R^{8'} und A für H oder eine Aminoschutzgruppe und R^{1'}, R^{3'}, R^{4'}, R^{5'} und R^{6'} für H stehen: sowie
b) R^{2'} für den Rest -COOH und A und R^{1'} für H, und ein oder zwei R^{3'}, R^{4'}, R^{5'} und R^{6'} für C₁-C₄-Alkyl stehen.

3. Substituierte Cyclohexan-1-yl-amin-Derivate der Formel (Ia) gemäß Anspruch 1, in welcher
R^{1'} für Wasserstoff, geradkettiges oder verzweigtes Alkyl mit 1 bis 4 Kohlenstoffatomen steht,
R^{2'} für Formyl, geradkettiges oder verzweigtes Hydroxyalkyl mit 1 bis 4 Kohlenstoffatomen im Alkylteil oder für einen der Reste 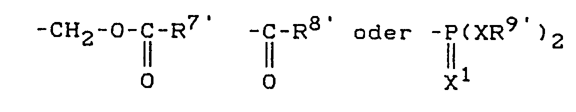 steht,
R^{3'} , R^{4'}, R^{5'} und R^{6'} gleich oder verschieden sind und jeweils für Wasserstoff, jeweils geradkettiges oder verzweigtes Alkyl oder Alkoxy mit 1 bis 6 Kohlenstoffatomen, Alkoxyalkyloxy mit jeweils 1 bis 6 Kohlenstoffatomen in den einzelnen Alkylteilen, für jeweils im Phenylteil unsubstituiertes oder einfach bis dreifach, gleich oder verschieden substituiertes Phenyl oder Phenylalkyl mit gegebenenfalls 1 oder 2 Kohlenstoffatomen im Alkylteil stehen, wobei als Phenylsubstituenten infrage kommen: Fluor, Chlor, Brom, Nitro, Cyano, Amino, C₁-C₂-Alkyl, C₁-C₃-Alkoxy, C₁-C₂-Alkylthio, Halogen-(C₁-C₂)-alkyl, Halogen-(C₁-C₂)-alkoxy, Halogen-(C₁-C₂)-alkylthio mit jeweils 1 bis 5 gleichen oder verschiedenen Fluor- und/ oder Chloratomen und Di-(C₁-C₂)-alkylamino, weiterhin für eine unsubstituierte oder einfach bis dreifach, gleich oder verschieden substituierte und gegebenenfalls über eine Methylengruppe gebundene heterocyclische fünf- bzw. sechsgliedrige Gruppierung aus der Reihe Furyl, Thienyl, Pyrrolyl, Pyrazolyl, Imidazolyl, 1,2,3- oder 1,2,4-Triazolyl, Oxazolyl, Isoxazolyl, 1,2,4- oder 1,3,4-Oxadiazolyl, Thiazolyl, Isothiazolyl, 1,2,3-, 1,2,4-, 1,2,5- oder 1,3,4-Thiadiazolyl, Pyridyl, Pyridazinyl, Pyrimidinyl und Pyrazinyl stehen, wobei als Substituenten für den Heterocyclus jeweils in Frage kommen: Fluor, Chlor, Brom, Nitro, Cyano, Amino, C₁-C₂-Alkyl, C₁-C₂-Alkoxy oder C₁-C₂-Alkylthio, Halogen-(C₁-C₂)-alkyl, Halogen-(C₁-C₂)-alkoxy, Halogen-(C₁-C₂)-alkylthio mit jeweils 1 bis 5 gleichen oder verschiedenen Fluor- und/oder Chloratomen und Di-(C₁-C₂)-alkylamino,
wobei mindestens zwei der Reste R^{3'}, R^{4'}, R^{5'} und R^{6'} für Wasserstoff stehen,
R^{7'} für jeweils geradkettiges oder verzweigtes Alkyl oder Alkoxy mit 1 bis 4 Kohlenstoffatomen steht,
R^{8'} für Hydroxy, geradkettiges oder verzweigtes Hydroxyalkyloxy mit 1 bis 6 Kohlenstoffatomen, geradkettiges oder verzweigtes Halogenalkyloxy mit 1 bis 6 Kohlenstoffatomen und 1 bis 13 gleichen oder verschiedenen Halogenatomen, jeweils unsubstituiertes oder einfach bis dreifach, gleich oder verschieden durch Fluor, Chlor, Brom substituiertes Alkenylalkyloxy, Alkinylalkyloxy und Cycloalkyloxy mit 3 bis 6 Kohlenstoffatomen, jeweils geradkettiges oder verzweigtes Alkoxy oder Alkylthio mit 1 bis 4 Kohlenstoffatomen, geradkettiges oder verzweigtes Alkoxyalkyloxy mit jeweils 1 bis 4 Kohlenstoffatomen im Alkoxy- oder Alkylteil, jeweils im Phenylteil unsubstituiertes oder einfach bis dreifach, gleich oder verschieden substituiertes Phenyloxy, Phenylthio, Phenylalkyl oder Phenylalkyloxy mit jeweils gegebenenfalls 1 bis 6 Kohlenstoffatomen im Alkylteil steht, wobei als Phenylsubstituenten die oben aufgeführten Phenylsubstituenten in Frage kommen
oder für eine Gruppe -O-Z-NR^{10'}R^{11'},
-NR^{10'}R^{11'} oder -OM steht,
R^{9'} für Wasserstoff oder geradkettiges oder verzweigtes Alkyl mit 1 bis 4 Kohlenstoffatomen steht,
R^{10'} und R^{11'} gleich oder verschieden sind und für Wasserstoff, geradkettiges oder verzweigtes Alkyl mit 1 bis 4 Kohlenstoffatomen oder unsubstituiertes oder einfach bis dreifach, gleich oder verschieden substituiertes Phenyl stehen, wobei als Phenylsubstituenten die oben aufgeführten Phenylsubstituenten in Frage kommen,
M für Wasserstoff oder für ein Äquivalent eines entsprechenden Alkalimetall-, Erdalkalimetall- oder Ammoniumkations steht und
Z für eine geradkettige oder verzweigte Alkylkette mit 1 bis 6 Kohlenstoffatomen steht
A für wasserstoff oder eine Aminoschutzgruppe steht und
X und X¹ gleich oder verschieden sind und für Sauerstoff oder Schwefel stehen,
sowie deren Säureadditions-Salze und Metallsalz-Komplexe, ausgenommen die Verbindung 2-Hydroxymethyl-cyclohexyl-1-amin und Verbindungen in denen
a) R^{2'} für den Rest -CO-R^{8'} und A für H oder eine Aminoschutzgruppe und R^{1'}, R^{3'}, R^{4'}, R^{5'} und R^{6'} für H stehen, sowie
b) R^{2'} für den Rest -COOH und A und R^{1'} für H, und ein oder zwei R^{3'}, R^{4'}, R^{5'} und R^{6'} für C₁-C₄-Alkyl stehen.

4. Verfahren zur Herstellung von substituierten Cyclo-hexan-1-yl-amin-Derivaten der Formel (Ia) 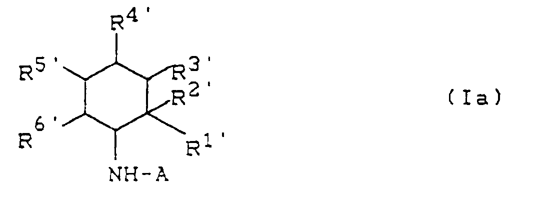 in welcher
R^{1'}, R^{2'}, R^{3'}, R^{4'}, R^{5'}, R^{6'} und A die in Anspruch 2 angegebene Bedeutung haben,
sowie deren Säureadditions-Salze und Metallsalz-Komplexe, dadurch gekennzeichnet, daß man
A) 2-Cyclohexen-1-yl-amin-Derivate der Formel (II) 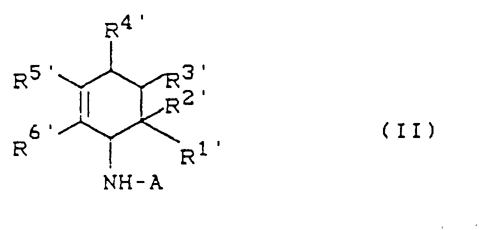 oder
B) Phenylamin-Derivate der Formel (III) 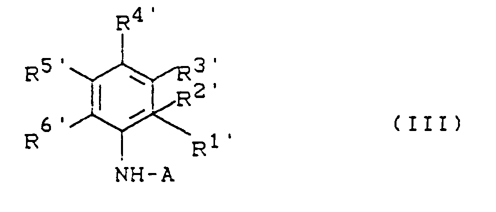 in welcher
R^{1'}, R^{2'}, R^{3'}, R^{4'}, R^{5'}, R^{6'} und A jeweils die oben angegebene Bedeutung haben und
in allgemein üblicher Weise, gegebenenfalls in Gegenwart eines Verdünnungsmittels und in Gegenwart eines Katalysators bei Temperaturen von 0°C bis 200°C, und Drücken von 10 bis 300 bar mit Wasserstoff hydriert, oder
C) aus den Cyclohexan-Derivaten der Formel (Ib) 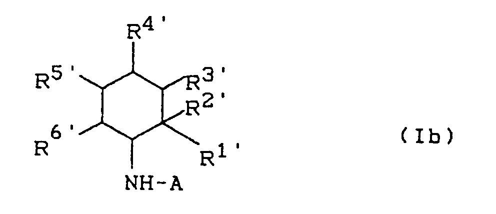 in welcher
R^{1'}, R^{2'}, R^{3'}, R^{4'}, R^{5'}, R^{6'} und A die oben angegebene Bedeutung haben und
die Aminoschutzgruppe,
in an sich bekannter Weise nach üblichen Methoden, gegebenenfalls in Gegenwart eines geeigneten Lösungs- oder Verdünnungsmittels oder eines Gemisches aus ihnen, unter Kühlen, bei Raumtemperatur oder unter Erwärmen und, falls erforderlich, in einem geschlossenen Gefäss, unter Druck, in einer Inertgasatmosphäre und/oder unter wasserfreien Bedingungen, abspaltet und die so erhaltenen Produkte gegebenenfalls in Säureadditions-Salze oder Metallsalz-Komplexe überführt.

5. Verwendung von Cyclohexan-1-yl-amin-Derivaten der Formel (Ia) nach den Ansprüchen 1 bis 3 zur Bekämpfung von Schädlingen.

6. Verfahren zur Bekämpfung von Schädlingen, dadurch gekennzeichnet, daß man Cyclohexan-1-yl-amin-Derivate der Formel (Ia) nach den Ansprüchen 1 bis 3 auf Schädlinge und/oder ihren Lebensraum einwirken läßt.

7. Verfahren zur Herstellung von Schädlingsbekämpfungsmitteln, dadurch gekennzeichnet, daß man Cyclohexan-1-yl-amin-Derivate der Formel (Ia) nach den Ansprüchen 1 bis 3 mit Streckmitteln und/oder oberflächenaktiven Mitteln vermischt.

## Claims

1. Use of the compounds of the formula (I) 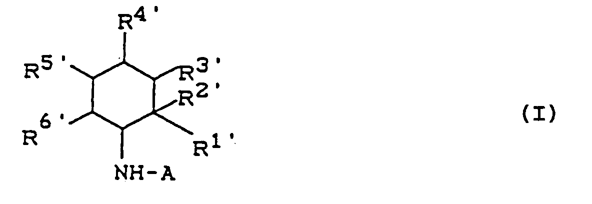 in which
R^{1'} represents hydrogen, halogen or straight-chain or branched alkyl having 1 to 6 carbon atoms,
R^{2'} represents formyl, straight-chain or branched hydroxyalkyl having 1 to 8 carbon atoms in the alkyl moiety, or represents one of the radicals 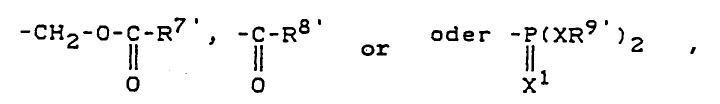
R^{3'}, R^{4'}, R^{5'} and R^{6'} are identical or different and in each case represent hydrogen, in each case straight-chain or branched alkyl or alkoxy having 1 to 8 carbon atoms, alkoxyalkyloxy having 1 to 8 carbon atoms in each of the individual alkyl moieties, or represent aryl or aralkyl, each of which has 6 to 10 carbon atoms in the aryl moiety and if appropriate 1 to 4 carbon atoms in the alkyl moiety and each of which is unsubstituted or monosubstituted to pentasubstituted in the aryl moiety by identical or different substituents, suitable aryl substituents being: halogen, nitro, cyano, amino, C₁-C₄-alkyl, C₁-C₄-alkoxy, C₁-C₄-alkylthio, halogeno-(C₁-C₄)-alkyl, halogeno-(C₁-C₄)-alkoxy, halogeno-(C₁-C₄)-alkylthio, each of which has 1 to 9 identical or different halogen atoms, and di-(C₁-C₄)-alkylamino, furthermore represent a heterocyclic 5- or 6-membered group from the series comprising furyl, thienyl, pyrrolyl, pyrazolyl, imidazolyl, 1,2,3- or 1,2,4-triazolyl, oxazolyl, isoxazolyl, 1,2,4-or 1,3,4-oxadiazolyl, thiazolyl, isothiazolyl, 1,2,3-, 1,2,4-, 1,2,5- or 1,3,4-thiadiazolyl, pyridyl, pyridazinyl, pyrimidinyl and pyrazinyl, each of which is bonded, if appropriate, via a methylene group and each of which is unsubstituted or monosubstituted to trisubstituted by identical or different substituents, suitable substituents for the heterocycle in each case being: halogen, nitro, cyano, amino, C₁-C₄-alkyl, C₁-C₄-alkoxy or C₁-C₄-alkylthio, halogeno-(C₁-C₄)-alkyl, halogeno-(C₁-C₄)-alkoxy or halogeno-(C₁-C₄)-alkylthio, each of which has 1 to 9 identical or different halogen atoms, and di-(C₁-C₄)-alkylamino,
where at least two of the radicals R^{3'}, R^{4'}, R^{5'} or R^{6'} represent hydrogen,
R^{7'} represents in each case straight-chain or branched alkyl or alkoxy having 1 to 6 carbon atoms,
R^{8'} represents hydroxyl, straight-chain or branched hydroxyalkyloxy having 1 to 8 carbon atoms, straight-chain or branched halogenoalkyloxy having 1 to 8 carbon atoms and 1 to 17 identical or different halogen atoms, or represents alkenyl-alkyloxy, alkinylalkyloxy and cycloalkyloxy, each of which has 3 to 6 carbon atoms and each of which is unsubstituted or monosubstituted to polysubstituted by identical or different halogen substituents, in each case straight-chain or branched alkoxy or alkylthio having 1 to 6 carbon atoms, or straight-chain or branched alkoxyalkyloxy having 1 to 6 carbon atoms in each of the alkoxy or alkyl moieties, or represents aryloxy, arylthio, aralkyl or aralkyloxy, each of which has 6 to 10 carbon atoms in the aryl moiety and if appropriate 1 to 8 carbon atoms in the alkyl moiety and each of which is unsubstituted or monosubstituted to pentasubstituted in the aryl moiety by identical or different substituents, suitable aryl substituents being the aryl substituents mentioned above, or represents a group -O-Z-NR^{10'}R^{11'}, -NR^{10'}R^{11'} or -OM,
R^{9'} represents hydrogen or straight-chain or branched alkyl having 1 to 6 carbon atoms,
R^{10'} and R^{11'} are identical or different and represent hydrogen, straight-chain or branched alkyl having 1 to 6 carbon atoms, or aryl which has 6 to 10 carbon atoms and which is unsubstituted or monosubstituted to pentasubstituted by identical or different substituents,
suitable aryl substituents being the aryl substituents mentioned above,
M represents hydrogen or represents an equivalent of a corresponding alkali metal cation, alkaline earth metal cation or ammonium cation and
Z represents a straight-chain or branched alkyl chain having 1 to 8 carbon atoms,
A represents hydrogen or an amino protective group, and
X and X¹ are identical or different and represent oxygen or sulphur,
and their acid addition salts and metal salt complexes,
as pesticides.

2. Substituted cyclohexan-1-yl-amine derivatives of the formula (Ia) 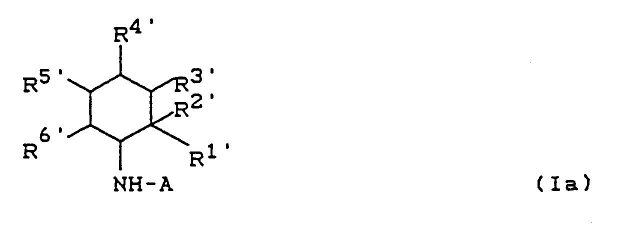 in which
R^{1'} represents hydrogen, halogen or straight-chain or branched alkyl having 1 to 6 carbon atoms,
R^{2'} represents formyl, straight-chain or branched hydroxyalkyl having 1 to 8 carbon atoms in the alkyl moiety, or represents one of the radicals 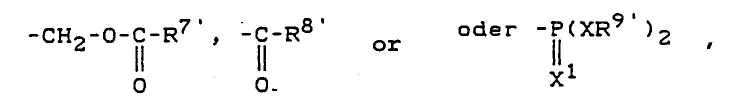
R^{3'}, R^{4'}, R^{5'} and R^{6'} are identical or different and in each case represent hydrogen, in each case straight-chain or branched alkyl or alkoxy having 1 to 8 carbon atoms, alkoxyalkyloxy having 1 to 8 carbon atoms in each of the individual alkyl moieties, or represent aryl or aralkyl, each of which has 6 to 10 carbon atoms in the aryl moiety and if appropriate 1 to 4 carbon atoms in the alkyl moiety and each of which is unsubstituted or monosubstituted to pentasubstituted in the aryl moiety by identical or different substituents, suitable aryl substituents being: halogen, nitro, cyano, amino, C₁-C₄-alkyl, C₁-C₄-alkoxy, C₁-C₄-alkylthio, halogeno-(C₁-C₄)-alkyl, halogeno-(C₁-C₄)-alkoxy, halogeno-(C₁-C₄)-alkylthio, each of which has 1 to 9 identical or different halogen atoms, and di-(C₁-C₄)-alkylamino, furthermore represent a heterocyclic 5- or 6-membered group from the series comprising furyl, thienyl, pyrrolyl, pyrazolyl, imidazolyl, 1,2,3- or 1,2,4-triazolyl, oxazolyl, isoxazolyl, 1,2,4- or 1,3,4-oxadiazolyl, thiazolyl, isothiazolyl, 1,2,3-, 1,2,4-, 1,2,5-or 1,3,4-thiadiazolyl, pyridyl, pyridazinyl, pyrimidinyl and pyrazinyl, each of which is bonded, if appropriate, via a methylene group and each of which is unsubstituted or monosubstituted to trisubstituted by identical or different substituents, suitable substituents for the heterocycle in each case being: halogen, nitro, cyano, amino, C₁-C₄-alkyl, C₁-C₄-alkoxy or C₁-C₄-alkylthio, halogeno-(C₁-C₄)-alkyl, halogeno-(C₁-C₄)-alkoxy or halogeno-(C₁-C₄)-alkylthio, each of which has 1 to 9 identical or different halogen atoms, and di-(C₁-C₄)-alkylamino,
where at least two of the radicals R^{3'}, R^{4'}, R^{5'} or R^{6'} represent hydrogen,
R^{7'} represents in each case straight-chain or branched alkyl or alkoxy having 1 to 6 carbon atoms,
R^{8'} represents hydroxyl, straight-chain or branched hydroxyalkyloxy having 1 to 8 carbon atoms,
straight-chain or branched halogenoalkyloxy having 1 to 8 carbon atoms and 1 to 17 identical or different halogen atoms, or represents alkenyl-alkyloxy, alkinylalkyloxy and cycloalkyloxy, each of which has 3 to 6 carbon atoms and each of which is unsubstituted or monosubstituted to polysubstituted by identical or different halogen substituents, in each case straight-chain or branched alkoxy or alkylthio having 1 to 6 carbon atoms, or straight-chain or branched alkoxyalkyloxy having 1 to 6 carbon atoms in each of the alkoxy or alkyl moieties, or represents aryloxy, arylthio, aralkyl or aralkyloxy, each of which has 6 to 10 carbon atoms in the aryl moiety and if appropriate 1 to 8 carbon atoms in the alkyl moiety and each of which is unsubstituted or monosubstituted to pentasubstituted in the aryl moiety by identical or different substituents, suitable aryl substituents being the aryl substituents mentioned above, or represents a group -O-Z-NR^{10'}R^{11'}, NR^{10'}R^{11'} or -OM,
R^{9'} represents hydrogen or straight-chain or branched alkyl having 1 to 6 carbon atoms,
R^{10'} and R^{11'} are identical or different and represent hydrogen, straight-chain or branched alkyl having 1 to 6 carbon atoms, or aryl which has 6 to 10 carbon atoms and which is unsubstituted or monosubstituted to pentasubstituted by identical or different substituents,
suitable aryl substituents being the aryl substituents mentioned above,
M represents hydrogen or represents an equivalent of a corresponding alkali metal cation, alkaline earth metal cation or ammonium cation and
Z represents a straight-chain or branched alkyl chain having 1 to 8 carbon atoms,
A represents hydrogen or an amino protective group, and
X and X¹ are identical or different and represent oxygen or sulphur,
and their acid addition salts and metal salt complexes, with the exception of the compound 2-hydroxymethyl-cyclohexyl-1-amine and compounds in which
a) R^{2'} represents the radical -CO-R^{8'} and A represents H or an amino protective group and R^{1'}, R^{3'}, R^{4'}, R^{5'} and R^{6'} represent H, and
b) R^{2'} represents the radical -COOH, and A and R^{1'} represent H, and one or two R^{3'}, R^{4'}, R^{5'} and R^{6'} represent C₁-C₄-alkyl.

3. Substituted cyclohexan-1-yl-amine derivatives of the formula (Ia) according to Claim 1, in which
R^{1'} represents hydrogen or straight-chain or branched alkyl having 1 to 4 carbon atoms,
R^{2'} represents formyl, straight-chain or branched hydroxyalkyl having 1 to 4 carbon atoms in the alkyl moiety, or represents one of the radicals 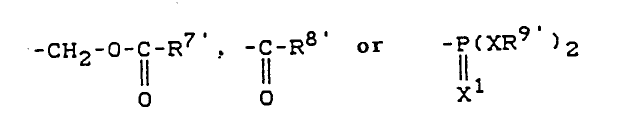
R^{3'}, R^{4'}, R^{5'} and R^{6'} are identical or different and in each case represent hydrogen, in each case straight-chain or branched alkyl or alkoxy having 1 to 6 carbon atoms, alkoxyalkyloxy having 1 to 6 carbon atoms in each of the individual alkyl moieties, or represent phenyl or phenylalkyl, having, where appropriate, 1 or 2 carbon atoms in the alkyl moiety, each of which is unsubstituted or monosubstituted to trisubstituted in the phenyl moiety by identical or different substituents, suitable phenyl substituents being: fluorine, chlorine, bromine, nitro, cyano, amino, C₁-C₂-alkyl, C₁-C₃-alkoxy or C₁-C₂-alkylthio, halogeno-(C₁-C₂)-alkyl, halogeno-(C₁-C₂)-alkoxy or halogeno-(C₁-C₂)-alkylthio, each of which has 1 to 5 identical or different fluorine and/or chlorine atoms, and di-(C₁-C₂)-alkylamino, furthermore represent a heterocyclic five- or six-membered group from the series comprising furyl, thienyl, pyrrolyl, pyrazolyl, imidazolyl, 1,2,3- or 1,2,4-triazolyl, oxazolyl, isoxazolyl, 1,2,4- or 1,3,4-oxadiazolyl, thiazolyl, isothiazolyl, 1,2,3-, 1,2,4-, 1,2,5- or 1,3,4-thiadiazolyl, pyridyl, pyridazinyl, pyrimidinyl and pyrazinyl, each of which is, if appropriate, bonded via a methylene group and each of which is unsubstituted or monosubstituted to trisubstituted by identical or different substituents, suitable substituents for the heterocycle in each case being: fluorine, chlorine, bromine, nitro, cyano, amino, C₁-C₂-alkyl, C₁-C₂-alkoxy or C₁-C₂-alkylthio, halogeno-(C₁-C₂)-alkyl, halogeno-(C₁-C₂)-alkoxy. halogeno-(C₁-C₂)-alkylthio, each of which has 1 to 5 identical or different fluorine and/or chlorine atoms, and di-(C₁-C₂)-alkylamino,
where at least two of the radicals R^{3'}, R^{4'}, R^{5'} and R^{6'} represent hydrogen,
R^{7'} represents in each case straight-chain or branched alkyl or alkoxy having 1 to 4 carbon atoms,
R^{8'} represents hydroxyl, straight-chain or branched hydroxyalkyloxy having 1 to 6 carbon atoms, straight-chain or branched halogenoalkyloxy having 1 to 6 carbon atoms and 1 to 13 identical or different halogen atoms, or represents alkenylalkyloxy. alkinylalkyloxy and cycloalkyloxy, each of which has 3 to 6 carbon atoms and each of which is unsubstituted or monosubstituted to trisubstituted by identical or different substituents from the series comprising fluorine, chlorine and bromine, or represents in each case straight-chain or branched alkoxy or alkylthio having 1 to 4 carbon atoms, straight-chain or branched alkoxyalkyloxy having 1 to 4 carbon atoms in each of the alkoxy or alkyl moieties, or represents phenyloxy, phenylthio, phenylalkyl or phenylalkyloxy, each of which has, if appropriate, 1 to 6 carbon atoms in the alkyl moiety and each of which is unsubstituted or monosubstituted to trisubstituted in the phenyl moiety by identical or different substituents, suitable phenyl substituents being the phenyl substituents mentioned above, or represents a group -O-Z-NR^{10'}R^{11'}, -NR^{10'}R^{11'} or -OM,
R^{9'} represents hydrogen or straight-chain or branched alkyl having 1 to 4 carbon atoms,
R^{10'} and R^{11'} are identical or different and represent hydrogen, straight-chain or branched alkyl having 1 to 4 carbon atoms, or represent phenyl which is unsubstituted or monosubstituted to trisubstituted by identical or different substituents, suitable phenyl substituents being the phenyl substituents mentioned above,
M represents hydrogen, or represents an equivalent of a corresponding alkali metal cation, alkaline earth metal cation or ammonium cation and
Z represents a straight-chain or branched alkyl chain having 1 to 6 carbon atoms
A represents hydrogen or an amino protective group, and
X and X¹ are identical or different and represent oxygen or sulphur,
and their acid addition salts and metal salt complexes with the exception of the compound 2-hydroxymethyl-cyclohexyl-1-amine and compounds in which
a) R^{2'} represents the radical -CO-R^{8'} and A represents H or an amino protective group and R^{1'}, R^{3'}, R^{4'}, R^{5'} and R^{6'} represent H, and
b) R^{2'} represents the radical -COOH, and A and R^{1'} represent H, and one or two R^{3'}, R^{4'}, R^{5'} and R^{6'} represent C₁-C₄-alkyl.

4. Process for the preparation of substituted cyclohexan-1-yl-amine derivatives of the formula (Ia) 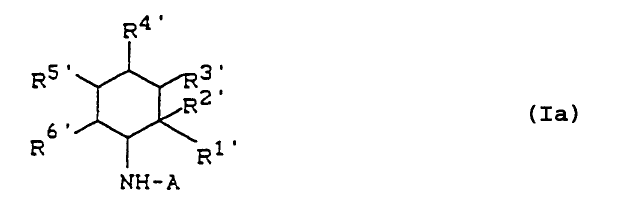 in which
R^{1'}, R^{2'}, R^{3'}, R^{4'}, R^{5'}, R^{6'} and A have the meaning given in Claim 2,
and their acid addition salts and metal salt complexes, characterised in that
A) 2-cyclohexan-1-yl-amine derivatives of the formula (II) 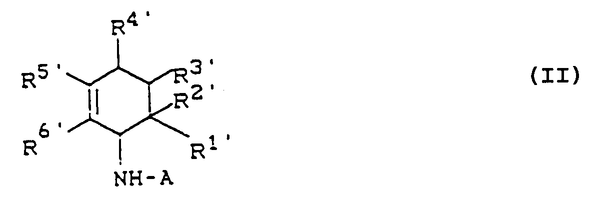 or
B) phenylamine derivatives of the formula (III) 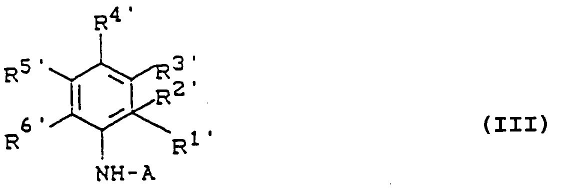 in which
R^{1'}, R^{2'}, R^{3'}, R^{4'}, R^{5'}, R^{6'} and A in each case have the abovementioned meaning
are hydrogenated in a generally customary manner with hydrogen at temperatures from 0°C to 200°C and pressures from 10 to 300 bar, if appropriate in the presence of a diluent and in the presence of a catalyst,
or
C) the amino protective group is eliminated from the cyclohexane derivatives of the formula (Ib) 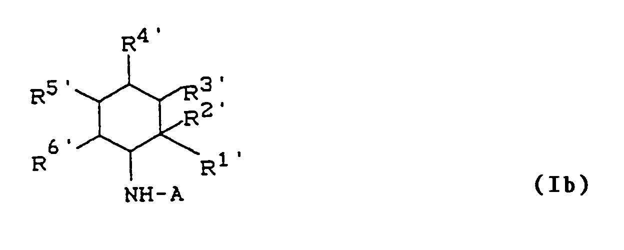 in which
R^{1'}, R^{2'}, R^{3'}, R^{4'}, R^{5'}, R^{6'} and A have the above-mentioned meaning
in a manner known per se by customary methods, if appropriate in the presence of a suitable solvent or diluent or a mixture of these, with cooling, at room temperature or with heating and, if required, in a sealed vessel, under pressure, in an inert-gas atmosphere and/or under anhydrous conditions, and, if desired, the resulting products are converted into acid addition salts or metal salt complexes.

5. Use of cyclohexan-1-yl-amine derivatives of the formula (Ia) according to Claims 1 to 3 for combating pests.

6. Process for combating pests, characterised in that cyclohexan-1-yl-amine derivatives of the formula (Ia) according to Claims 1 to 3 are allowed to act on pests and/or their environment.

7. Process for the preparation of pesticides, characterised in that cyclohexan-1-yl-amine derivatives of the formula (Ia) according to Claims 1 to 5 are mixed with extenders and/or surface-active agents.

## Revendications

1. Utilisation des composés répondant à la formule (Ia) 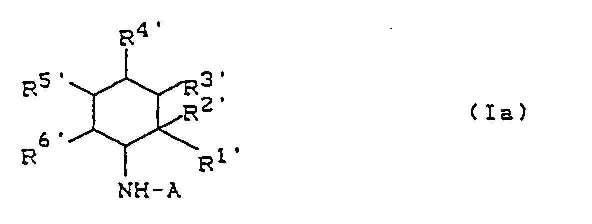 dans laquelle
R^{1'} représente un atome d'hydrogène, un atome d'halogène ou un groupe alkyle à chaîne droite ou ramifiée contenant de 1 à 6 atomes de carbone,
R^{2'} représente un groupe formyle, un groupe hydroxyalkyle à chaîne droite ou ramifiée contenant de 1 à 8 atomes de carbone dans la fraction alkyle ou encore un des radicaux 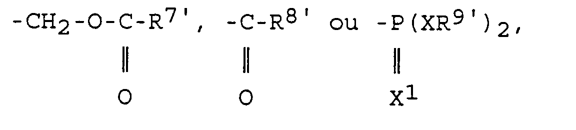
R^{3'}, R^{4'}, R^{5'} et R^{6'} sont identiques ou différents et représentent respectivement un atome d'hydrogène; un groupe alkyle ou un groupe alcoxy contenant de 1 à 8 atomes de carbone, un groupe alcoxyalkyloxy contenant respectivement de 1 à 8 atomes de carbone dans les fractions alkyle individuelles, chacun de ces groupes étant à chaîne droite ou ramifiée; un groupe aryle ou un groupe aralkyle contenant respectivement de 6 à 10 atomes de carbone dans la fraction aryle et éventuellement de 1 à 4 atomes de carbone dans la fraction alkyle, chacun de ces groupes étant non substitué ou substitué de une à cinq fois de manière identique ou différente dans la fraction aryle, dans lesquels, comme substituants du groupe aryle, on envisage: un atome d'halogène, un groupe nitro, un groupe cyano, un groupe amino, un groupe alkyle en C₁-C₄, un groupe alcoxy en C₁-C₄, un groupe alkyl(en C₁-C₄)thio, un groupe halogénalkyle en C₁-C₄, un groupe halogénalcoxy en C₁-C₄, un groupe halogénalkyl(en C₁-C₄)thio contenant respectivement de 1 à 9 atomes d'halogène identiques ou différents et un groupe dialkyl(en C₁-C₄)amino;
représentent en outre un groupement hétérocyclique penta- ou hexagonal non substitué ou substitué de une a trois fois de manière identique ou différente et éventuellement lié via un groupe méthylène, choisi parmi la série comprenant un groupe furyle, un groupe thiényle, un groupe pyrrolyle, un groupe pyrazolyle, un groupe imidazolyle, un groupe 1,2,3- ou 1,2,4-triazolyle, un groupe oxazolyle, un groupe isoxazolyle, un groupe 1,2,4- ou 1,3,4-oxadiazolyle, un groupe thiazolyle, un groupe isothiazolyle, un groupe 1,2,3-, 1,2,4-, 1,2,5- ou 1,3,4-thiadiazolyle, un groupe pyridyle, un groupe pyridazinyle, un groupe pyrimidinyle et un groupe pyrazinyle, dans lequel, comme substituants pour le radical hétérocyclique, on envisage respectivement: un atome d'halogène, un groupe nitro, un groupe cyano, un groupe amino, un groupe alkyle en C₁-C₄, un groupe alcoxy en C₁-C₄ ou un groupe alkyl(en C₁-C₄)thio, un groupe halogénalkyle en C₁-C₄, un groupe halogénalcoxy en C₁-C₄, un groupe halogénalkyl(en C₁-C₄)thio contenant respectivement de 1 à 9 atomes d'halogène identiques ou différents et un groupe dialkyl(en C₁-C₄)amino,
au moins deux des radicaux R^{3'}, R^{4'}, R^{5'} ou R^{6'} représentant un atome d'hydrogène,
R^{7'} représente un groupe alkyle ou un groupe alcoxy contenant de 1 à 6 atomes de carbone, chacun de ces groupes étant à chaîne droite ou ramifiée,
R^{8'} représente un groupe hydroxyle; un groupe hydroxyalkyloxy à chaîne droite ou ramifiée contenant de 1 à 8 atomes de carbone; un groupe halogénalkyloxy à chaîne droite ou ramifiée contenant de 1 à 8 atomes de carbone et de 1 à 17 atomes d'halogène identiques ou différents; un groupe alcénylalkyloxy, un groupe alcynylalkyloxy et un groupe cycloalkyloxy contenant de 3 à 6 atomes de carbone, chacun de ces groupes étant non substitué ou substitué de une à plusieurs fois de manière identique ou différente par un atome d'halogène; un groupe alcoxy ou un groupe alkylthio contenant de 1 à 6 atomes de carbone, chacun de ces groupes étant à chaîne droite ou ramifiée; un groupe alcoxyalkyloxy à chaîne droite ou ramifiée contenant respectivement de 1 à 6 atomes de carbone dans la fraction alcoxy ou alkyle; un groupe aryloxy, un groupe arylthio, un groupe aralkyle ou un groupe aralkyloxy contenant respectivement de 6 à 10 atomes de carbone dans la fraction aryle et éventuellement de 1 à 8 atomes de carbone dans la fraction alkyle, chacun de ces groupes étant non substitué ou substitué de une a cinq fois de manière identique ou différente dans la fraction aryle, dans lesquels, comme substituants du groupe aryle, on envisage les substituants du groupe aryle repris ci-dessus; ou encore un groupe -O-Z-NR^{10'}R^{11'}, -NR^{10'}R^{11'} ou -OM,
R^{9'} représente un atome d'hydrogène ou un groupe alkyle à chaîne droite ou ramifiée contenant de 1 à 6 atomes de carbone,
R^{10'} et R^{11'} sont identiques ou différents et représentent un atome d'hydrogène; un groupe alkyle à chaîne droite ou ramifiée contenant de 1 à 6 atomes de carbone; ou encore un groupe aryle contenant de 6 à 10 atomes de carbone, non substitué ou substitué de une à cinq fois de manière identique ou différente,
dans lequel, comme substituants du groupe aryle, on envisage les substituants du groupe aryle repris ci-dessus,
M représente un atome d'hydrogène ou un équivalent d'un cation correspondant de métal alcalin, de métal alcalino-terreux ou d'ammonium, et
Z représente une chaîne alkyle droite ou ramifiée contenant de 1 à 8 atomes de carbone,
A représente un atome d'hydrogène ou un groupe protecteur du groupe amino, et
X et X¹ sont identiques ou différents et représentent un atome d'oxygène ou un atome de soufre,
ainsi que de leurs sels d'addition d'acides et de leurs complexes de sels métalliques,
comme agents de lutte contre les parasites.

2. Dérivés substitués de cyclohexylamine répondant à la formule (Ia) 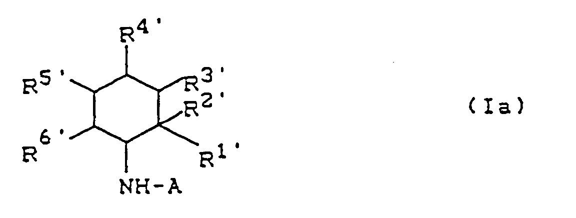 dans laquelle
R^{1'} représente un atome d'hydrogène, un atome d'halogène ou un groupe alkyle à chaîne droite ou ramifiée contenant de 1 à 6 atomes de carbone,
R^{2'} représente un groupe formyle, un groupe hydroxyalkyle à chaîne droite ou ramifiée contenant de 1 à 8 atomes de carbone dans la fraction alkyle ou encore un des radicaux 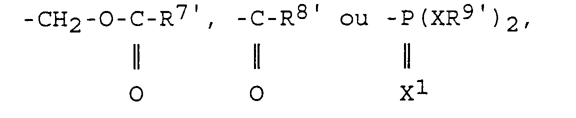
R^{3'}, R^{4'}, R^{5'} et R^{6'} sont identiques ou différents et représentent respectivement un atome d'hydrogène; un groupe alkyle ou un groupe alcoxy contenant de 1 à 8 atomes de carbone, un groupe alcoxyalkyloxy contenant respectivement de 1 à 8 atomes de carbone dans les fractions alkyle individuelles, chacun de ces groupes étant à chaîne droite ou ramifiée; un groupe aryle ou un groupe aralkyle contenant respectivement de 6 à 10 atomes de carbone dans la fraction aryle et éventuellement de 1 à 4 atomes de carbone dans la fraction alkyle, chacun de ces groupes étant non substitué ou substitué de une à cinq fois de manière identique ou différente dans la fraction aryle, dans lesquels, comme substituants du groupe aryle, on envisage: un atome d'halogène, un groupe nitro, un groupe cyano, un groupe amino, un groupe alkyle en C₁-C₄, un groupe alcoxy en C₁-C₄, un groupe alkyl(en C₁-C₄)thio, un groupe halogénalkyle en C₁-C₄, un groupe halogénalcoxy en C₁-C₄, un groupe halogénalkyl(en C₁-C₄)thio contenant respectivement de 1 à 9 atomes d'halogène identiques ou différents et un groupe dialkyl(en C₁-C₄)amino;
représentent en outre un groupement hétérocyclique penta- ou hexagonal non substitué ou substitué de une à trois fois de manière identique ou différente et éventuellement lié via un groupe méthylène, choisi parmi la série comprenant un groupe furyle, un groupe thiényle, un groupe pyrrolyle, un groupe pyrazolyle, un groupe imidazolyle, un groupe 1,2,3- ou 1,2,4-triazolyle, un groupe oxazolyle, un groupe isoxazolyle, un groupe 1,2,4- ou 1,3,4-oxadiazolyle, un groupe thiazolyle, un groupe isothiazolyle, un groupe 1,2,3-, 1,2,4-, 1,2,5- ou 1,3,4-thiadiazolyle, un groupe pyridyle, un groupe pyridazinyle, un groupe pyrimidinyle et un groupe pyrazinyle, dans lequel, comme substituants pour le radical hétérocyclique, on envisage respectivement: un atome d'halogène, un groupe nitro, un groupe cyano, un groupe amino, un groupe alkyle en C₁-C₄, un groupe alcoxy en C₁-C₄ ou un groupe alkyl(en C₁-C₄)thio, un groupe halogénalkyle en C₁-C₄, un groupe halogénalcoxy en C₁-C₄, un groupe halogénalkyl(en C₁-C₄)thio contenant respectivement de 1 à 9 atomes d'halogène identiques ou différents et un groupe dialkyl(en C₁-C₄)amino,
au moins deux des radicaux R^{3'}, R^{4'}, R^{5'} ou R^{6'} représentant un atome d'hydrogène,
R^{7'} représente un groupe alkyle ou un groupe alcoxy contenant de 1 à 6 atomes de carbone, chacun de ces groupes étant à chaîne droite ou ramifiée,
R^{8'} représente un groupe hydroxyle; un groupe hydroxyalkyloxy à chaîne droite ou ramifiée contenant de 1 à 8 atomes de carbone; un groupe halogénalkyloxy à chaîne droite ou ramifiée contenant de 1 à 8 atomes de carbone et de 1 à 17 atomes d'halogène identiques ou différents; un groupe alcénylalkyloxy, un groupe alcynylalkyloxy et un groupe cycloalkyloxy contenant de 3 à 6 atomes de carbone, chacun de ces groupes étant non substitué ou substitué de une à plusieurs fois de manière identique ou différente par un atome d'halogène; un groupe alcoxy ou un groupe alkylthio contenant de 1 à 6 atomes de carbone, chacun de ces groupes étant à chaîne droite ou ramifiée; un groupe alcoxyalkyloxy à chaîne droite ou ramifiée contenant respectivement de 1 à 6 atomes de carbone dans la fraction alcoxy ou alkyle; un groupe aryloxy, un groupe arylthio, un groupe aralkyle ou un groupe aralkyloxy contenant respectivement de 6 à 10 atomes de carbone dans la fraction aryle et éventuellement de 1 à 8 atomes de carbone dans la fraction alkyle, chacun de ces groupes étant non substitué ou substitué de une a cinq fois de manière identique ou différente dans la fraction aryle, dans lesquels, comme substituants du groupe aryle, on envisage les substituants du groupe aryle repris ci-dessus; ou encore un groupe -O-Z-NR^{10'}R^{11'}, -NR^{10'}R^{11'} ou -OM,
R^{9'} représente un atome d'hydrogène ou un groupe alkyle à chaîne droite ou ramifiée contenant de 1 à 6 atomes de carbone,
R^{10'} et R^{11'} sont identiques ou différents et représentent un atome d'hydrogène; un groupe alkyle à chaîne droite ou ramifiée contenant de 1 à 6 atomes de carbone; ou encore un groupe aryle contenant de 6 à 10 atomes de carbone, non substitué ou substitué de une à cinq fois de manière identique ou différente,
dans lequel, comme substituants du groupe aryle, on envisage les substituants du groupe aryle repris ci-dessus,
M représente un atome d'hydrogène ou un équivalent d'un cation correspondant de métal alcalin, de métal alcalino-terreux ou d'ammonium, et
Z représente une chaîne alkyle droite ou ramifiée contenant de 1 à 8 atomes de carbone,
A représente un atome d'hydrogène ou un groupe protecteur du groupe amino, et
X et X¹ sont identiques ou différents et représentent un atome d'oxygène ou un atome de soufre,
ainsi que leurs sels d'addition d'acides et leurs complexes de sels métalliques, à l'exception du composé 2-hydroxyméthyl-cyclohexyl-1-amine et des composés dans lesquels
a) R^{2'} représente le radical -CO-R^{8'} et A représente H ou un groupe protecteur du groupe amino, et R^{1'}, R^{3'}, R^{4'}, R^{5'} et R^{6'} représentent H, ainsi que
b) R^{2'} représente le radical -COOH, et A et R^{1'} représentent H, un ou deux des radicaux R^{3'}, R^{4'}, R^{5'} et R^{6'} représentant un groupe alkyle en C₁-C₄.

3. Dérivés substitués de cyclohexylamine de formule (Ia) selon la revendication 1, dans lesquels
R^{1'} représente un atome d'hydrogène; un groupe alkyle à chaîne droite ou ramifiée contenant de 1 à 4 atomes de carbone,
R^{2'} représente un groupe formyle; un groupe hydroxyalkyle à chaîne droite ou ramifiée contenant de 1 à 4 atomes de carbone dans la fraction alkyle ou encore un des radicaux 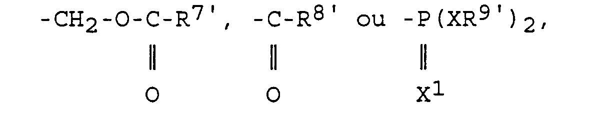
R^{3'}, R^{4'}, R^{5'} et R^{6'} sont identiques ou différents et représentent respectivement un atome d'hydrogène; un groupe alkyle ou un groupe alcoxy contenant de 1 à 6 atomes de carbone, chacun de ces groupes étant à chaîne droite ou ramifiée; un groupe alcoxyalkyloxy contenant respectivement de 1 à 6 atomes de carbone dans les fractions alkyle individuelles; un groupe phényle ou un groupe phénylalkyle contenant éventuellement 1 ou 2 atomes de carbone dans la fraction alkyle, chacun de ces groupes étant non substitué ou substitué de 1 à 3 fois de manière identique ou différente dans la fraction phényle, dans lesquels, comme substituants du groupe phényle, on envisage: un atome de fluor, un atome de chlore, un atome de brome, un groupe nitro, un groupe cyano, un groupe amino, un groupe alkyle en C₁-C₂, un groupe alcoxy en C₁-C₃, un groupe alkyl(en C₁-C₂)thio, un groupe halogénalkyle en C₁-C₂, un groupe halogénalcoxy en C₁-C₂, un groupe halogénalkyl(en C₁-C₂)thio contenant respectivement de 1 à 5 atomes de fluor et/ou de chlore identiques ou différents, et un groupe dialkyl(en C₁-C₂)amino;
représentent en outre un groupement hétérocyclique penta-, respectivement hexagonal non substitué ou substitué de une à trois fois de manière identique ou différente et éventuellement lié via un groupe méthylène, choisi parmi la série comprenant un groupe furyle, un groupe thiényle, un groupe pyrrolyle, un groupe pyrazolyle, un groupe imidazolyle, un groupe 1,2,3- ou 1,2,4-triazolyle, un groupe oxazolyle, un groupe isoxazolyle, un groupe 1,2,4- ou 1,3,4-oxadiazolyle, un groupe thiazolyle, un groupe isothiazolyle, un groupe 1,2,3-, 1,2,4-, 1,2,5- ou 1,3,4-thiadiazolyle, un groupe pyridyle, un groupe pyridazinyle, un groupe pyrimidinyle et un groupe pyrazinyle, dans lequel, comme substituants pour le radical hétérocyclique, on envisage respectivement: un atome de fluor, un atome de chlore, un atome de brome, un groupe nitro, un groupe cyano, un groupe amino, un groupe alkyle en C₁-C₂, un groupe alcoxy en C₁-C₂ ou un groupe alkyl(en C₁-C₂)thio, un groupe halogénalkyle en C₁-C₂, un groupe halogénalcoxy en C₁-C₂, un groupe halogénalkyl(en C₁-C₂)thio contenant respectivement de 1 à 5 atomes de fluor et/ou de chlore identiques ou différents, et un groupe dialkyl(en C₁-C₂)amino,
au moins deux des radicaux R3', R4', R5' et R6' représentant un atome d'hydrogène,
R^{7'} représente un groupe alkyle ou un groupe alcoxy contenant de 1 à 4 atomes de carbone, chacun de ces groupes étant à chaîne droite ou ramifiée,
R^{8'} représente un groupe hydroxyle; un groupe hydroxyalkyloxy à chaîne droite ou ramifiée contenant de 1 à 6 atomes de carbone; un groupe halogénalkyloxy à chaîne droite ou ramifiée contenant de 1 à 6 atomes de carbone et de 1 à 13 atomes d'halogène identiques ou différents; un groupe alcénylalkyloxy, un groupe alcynylalkyloxy et un groupe cycloalkyloxy contenant de 3 à 6 atomes de carbone, chacun de ces groupes étant non substitué ou substitué de une à trois fois de manière identique ou différente par un atome de fluor, par un atome de chlore, par un atome de brome; un groupe alcoxy ou un groupe alkylthio contenant de 1 à 4 atomes de carbone, chacun de ces groupes étant à chaîne droite ou ramifiée; un groupe alcoxyalkyloxy à chaîne droite ou ramifiée contenant respectivement de 1 à 4 atomes de carbone dans la fraction alcoxy ou alkyle; un groupe phényloxy, un groupe phénylthio, un groupe phénylalkyle ou un groupe phénylalkyloxy contenant respectivement, le cas échéant, de 1 à 6 atomes de carbone dans la fraction alkyle, chacun de ces groupes étant non substitué ou substitué de une a trois fois de manière identique ou différente dans la fraction phényle, dans lesquels, comme substituants du groupe phényle, on envisage les substituants du groupe phényle repris ci-dessus;
ou représente un groupe -O-Z-NR^{10'}R^{11'}, -NR^{10'}R^{11'} ou -OM,
R^{9'} représente un atome d'hydrogène ou un groupe alkyle à chaîne droite ou ramifiée contenant de 1 à 4 atomes de carbone,
R^{10'} et R^{11'} sont identiques ou différents et représentent un atome d'hydrogène; un groupe alkyle à chaîne droite ou ramifiée contenant de 1 à 4 atomes de carbone; ou encore un groupe phényle non substitué ou substitué de une à trois fois de manière identique ou différente, dans lequel, comme substituants du groupe phényle, on envisage les substituants du groupe phényle repris ci-dessus,
M représente un atome d'hydrogène ou un équivalent d'un cation correspondant de métal alcalin, de métal alcalino-terreux ou d'ammonium, et
Z représente une chaîne alkyle droite ou ramifiée contenant de 1 à 6 atomes de carbone,
A représente un atome d'hydrogène ou un groupe protecteur du groupe amino, et
X et X¹ sont identiques ou différents et représentent un atome d'oxygène ou un atome de soufre,
ainsi que leurs sels d'addition d'acides et leurs complexes de sels métalliques, à l'exception du composé 2-hydroxyméthyl-cyclohexyl-1-amine et des composés dans lesquels
a) R^{2'} représente le radical -CO-R^{8'} et A représente H ou un groupe protecteur du groupe amino, et R^{1'}, R^{3'}, R^{4'}, R^{5'} et R^{6'} représentent H, ainsi que
b) R^{2'} représente le radical -COOH, et A et R^{1'} représentent H, un ou deux des radicaux R^{3'}, R^{4'}, R^{5'} et R^{6'} représentant un groupe alkyle en C₁-C₄.

4. Procédé pour la préparation de dérivés substitués de cyclohexylamine répondant à la formule (Ia) 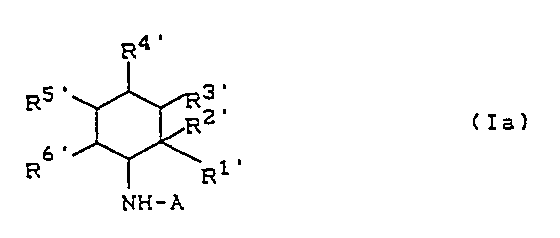 dans laquelle
R^{1'}, R^{2'}, R^{3'}, R^{4'}, R^{5'}, R^{6'} et A ont la signification indiquée à la revendication 2,
ainsi que de leurs sels d'addition d'acides et de leurs complexes de sels métalliques, caractérisé en ce que l'on soumet à une hydrogénation avec de l'hydrogène
A) des dérivés de 2-cylohexén-1-yl-amine répondant à la formule (II) 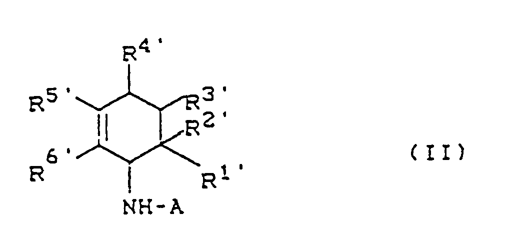 ou
B) des dérivés de phénylamine répondant à la formule (III) 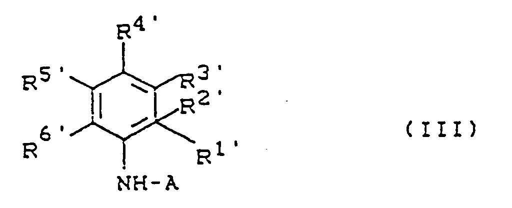 dans lesquelles
R^{1'}, R^{2'}, R^{3'}, R^{4'}, R^{5'}, R^{6'} et A ont respectivement la signification indiquée ci-dessus,
d'une manière généralement habituelle, le cas échéant, en présence d'un diluant et en présence d'un catalyseur, à des températures de 0°C à 200°C et sous des pressions de 10 à 300 bar, ou
C) on élimine des dérivés de cyclohexane de formule (Ib) 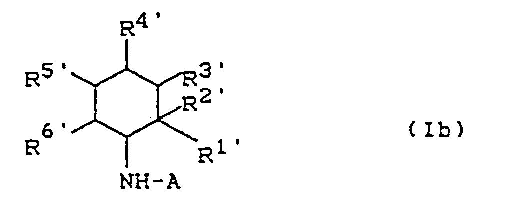 dans laquelle
R^{1'}, R^{2'}, R^{3'}, R^{4'}, R^{5'}, R^{6'} et A ont la signification indiquée ci-dessus,
le groupe protecteur du groupe amino,
d'une manière connue en soi, conformément à des procédés habituels, le cas échéant, en présence d'un solvant ou d'un diluant approprié ou encore d'un mélange de ces derniers, en refroidissant, à la température ambiante ou en réchauffant, et en cas de nécessité, dans un récipient fermé, sous pression, sous l'atmosphère d'un gaz inerte et/ou dans des conditions anhydres, et on transforme les produits ainsi obtenus, le cas échéant, en sels d'addition d'acides ou en complexes de sels métalliques.

5. Utilisation de dérivés de cyclohexylamine de formule (Ia) selon les revendications 1 à 3 pour lutter contre des parasites.

6. Procédé pour lutter contre des parasites, caractérisé en ce qu'on laisse agir des dérivés de cyclohexylamine de formule (Ia) selon les revendications 1 à 3 sur des parasites et/ou sur leur biotope.

7. Procédé pour la préparation d'agents de lutte contre les parasites, caractérisé en ce qu'on mélange des dérivés de cyclohexylamine de formule (Ia) selon les revendications 1 à 3 avec des diluants et/ou avec des agents tensioactifs.
